(19) Europäisches Patentamt
European Patent Office
Office européen des brevets

(11) EP 2 383 572 A1

(12) **EUROPEAN PATENT APPLICATION**

(43) Date of publication:
**02.11.2011 Bulletin 2011/44**

(51) Int Cl.:
***G01N 33/50*** *(2006.01)* ***G01N 33/564*** *(2006.01)*
***G01N 33/566*** *(2006.01)*

(21) Application number: **10358003.1**

(22) Date of filing: **28.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicants:
- **Assistance Publique Hôpitaux de Marseille**
  **13354 Marseille Cedex 5 (FR)**
- **Université de la Méditerranée (Aix-Marseille II)**
  **13284 Marseille Cédex 07 (FR)**
- **Etablissement Francais du Sang**
  **75015 Paris (FR)**

(72) Inventors:
- **Paul, Pascale**
  **13007 Marseille (FR)**
- **Picard, Christophe**
  **13013 Marseille (FR)**
- **Legris, Tristan**
  **13007 Marseille (FR)**
- **Dignat-George, Françoise**
  **13008 Marseille (FR)**

(74) Representative: **Barbot, Willy**
**SIMODORO**
**Parc Cézanne II, Bât. G**
**290 Avenue Galilée**
**13857 Aix en Provence Cedex 3 (FR)**

Remarks:
If details are not fully discernible,this is due to the quality of the drawings filed.The clearness of the drawings is the applicant's responsibility.

# (54) Cellular humoral activation test (CHAT)

(57) The present invention concerns an *in vitro* method for determining a subject cellular humoral activity, which comprises the step of (ii) determining the expression profile of at least the CX3CR1 gene in effector cells expressing FCRG and CX3CR1, preferably $CD3^-CD56^+$ NK cells, and (iii) comparing said expression profile to at least one control such as control values of FCRG and CX3CR1 expressing subsets in representative healthy donor control cohorts; the associated kit and the use of said kit for determining a subject cellular humoral activity directed against an antigen.

EP 2 383 572 A1

SEQUENCE LISTING

<110> Assistance publique-Hopitaux de Marseille
Université de la méditérranée
Etablissement Français du Sang
PAUL, Pascale
PICARD, Christophe
LEGRIS, Tristan
DIGNAT-GEORGE, Françoise

<120> Cellular Humoral Activation Test (CHAT)

<130> PRO-B-0001EP1

<160> 41

<170> PatentIn version 3.5

<210> 1
<211> 3108
<212> DNA
<213> Homo sapiens

<400> 1
gaaatactcg tctctggtaa agtctgagca ggacagggtg gctgactggc agatccagag 60
gttcccttgg cagtccacgc caggccttca ccatggatca gttccctgaa tcagtgacag 120
aaaactttga gtacgatgat ttggctgagg cctgttatat tggggacatc gtggtctttg 180
ggactgtgtt cctgtccata ttctactccg tcatctttgc cattggcctg gtgggaaatt 240
tgttggtagt gtttgccctc accaacagca agaagcccaa gagtgtcacc gacatttacc 300
tcctgaacct ggccttgtct gatctgctgt ttgtagccac tttgcccttc tggactcact 360
atttgataaa tgaaaagggc ctccacaatg ccatgtgcaa attcactacc gccttcttct 420
tcatcggctt ttttggaagc atattcttca tcaccgtcat cagcattgat aggtacctgg 480
ccatcgtcct ggccgccaac tccatgaaca accggaccgt gcagcatggc gtcaccatca 540
gcctaggcgt ctgggcagca gccattttgg tggcagcacc ccagttcatg ttcacaaagc 600
agaaagaaaa tgaatgcctt ggtgactacc ccgaggtcct ccaggaaatc tggcccgtgc 660
tccgcaatgt ggaaacaaat tttcttggct tcctactccc cctgctcatt atgagttatt 720
gctacttcag aatcatccag acgctgtttt cctgcaagaa ccacaagaaa gccaaagcca 780
ttaaactgat ccttctggtg gtcatcgtgt ttttcctctt ctggacaccc tacaacgtta 840
tgattttcct ggagacgctt aagctctatg acttctttcc cagttgtgac atgaggaagg 900
atctgaggct ggccctcagt gtgactgaga cggttgcatt tagccattgt tgcctgaatc 960
ctctcatcta tgcatttgct ggggagaagt tcagaagata cctttaccac ctgtatggga 1020
aatgcctggc tgtcctgtgt gggcgctcag tccacgttga tttctcctca tctgaatcac 1080
aaaggagcag gcatggaagt gttctgagca gcaattttac ttaccacacg agtgatggag 1140
atgcattgct ccttctctga agggaatccc aaagccttgt gtctacagag aacctggagt 1200
tcctgaacct gatgctgact agtgaggaaa gatttttgtt gttatttctt acaggcacaa 1260
aatgatggac ccaatgcaca caaaacaacc ctagagtgtt gttgagaatt gtgctcaaaa 1320

```
tttgaagaat gaacaaattg aactctttga atgacaaaga gtagacattt ctcttactgc     1380
aaatgtcatc agaacttttt ggtttgcaga tgacaaaaat tcaactcaga ctagtttagt     1440
taaatgaggg tggtgaatat tgttcatatt gtggcacaag caaaagggtg tctgagccct     1500
caaagtgagg ggaaaccagg gcctgagcca agctagaatt ccctctctct gactctcaaa     1560
tcttttagtc attatagatc ccccagactt tacatgacac agctttatca ccagagaggg     1620
actgacaccc atgtttctct ggccccaagg gcaaaattcc cagggaagtg ctctgatagg     1680
ccaagtttgt atcaggtgcc catccctgga aggtgctgtt atccatgggg aagggatata     1740
taagatggaa gcttccagtc caatctcatg gagaagcaga aatacatatt tccaagaagt     1800
tggatgggtg ggtactattc tgattacaca aaacaaatgc cacacatcac ccttaccatg     1860
tgcctgatcc agcctctccc ctgattacac cagcctcgtc ttcattaagc cctcttccat     1920
catgtcccca aacctgcaag ggctccccac tgcctactgc atcgagtcaa aactcaaatg     1980
cttggcttct catacgtcca ccatggggtc ctaccaatag attccccatt gcctcctcct     2040
tcccaaagga ctccacccat cctatcagcc tgtctcttcc atatgacctc atgcatctcc     2100
acctgctccc aggccagtaa gggaaataga aaaaccctgc ccccaaataa gaagggatgg     2160
attccaaccc caactccagt agcttgggac aaatcaagct tcagtttcct ggtctgtaga     2220
agagggataa ggtacctttc acatagagat catcctttcc agcatgagga actagccacc     2280
aactcttgca ggtctcaacc cttttgtctg cctcttagac ttctgctttc cacacctggc     2340
actgctgtgc tgtgcccaag ttgtggtgct gacaaagctt ggaagagcct gcaggtgctg     2400
ctgcgtggca tagcccagac acagaagagg ctggttctta cgatggcacc cagtgagcac     2460
tcccaagtct acagagtgat agccttccgt aacccaactc tcctggactg ccttgaatat     2520
cccctcccag tcaccttgtg gcaagcccct gcccatctgg gaaaataccc catcattcat     2580
gctactgcca acctggggag ccagggctat gggagcagct ttttttccc ccctagaaac     2640
gtttggaaca atctaaaagt ttaaagctcg aaaacaattg taataatgct aaagaaaaag     2700
tcatccaatc taaccacatc aatattgtca ttcctgtatt cacccgtcca gaccttgttc     2760
acactctcac atgtttagag ttgcaatcgt aatgtacaga tggttttata atctgatttg     2820
ttttcctctt aacgttagac cacaaatagt gctcgctttc tatgtagttt ggtaattatc     2880
attttagaag actctaccag actgtgtatt cattgaagtc agatgtggta actgttaaat     2940
tgctgtgtat ctgatagctc tttggcagtc tatatgtttg tataatgaat gagagaataa     3000
gtcatgttcc ttcaagatca tgtaccccaa tttacttgcc attactcaat tgataaacat     3060
ttaacttgtt tccaatgttt agcaaataca tattttatag aacttcca                  3108
```

<210> 2
<211> 355
<212> PRT
<213> Homo sapiens

<400> 2

```
Met Asp Gln Phe Pro Glu Ser Val Thr Glu Asn Phe Glu Tyr Asp Asp
1               5                   10                  15

Leu Ala Glu Ala Cys Tyr Ile Gly Asp Ile Val Val Phe Gly Thr Val
            20                  25                  30

Phe Leu Ser Ile Phe Tyr Ser Val Ile Phe Ala Ile Gly Leu Val Gly
        35                  40                  45

Asn Leu Leu Val Val Phe Ala Leu Thr Asn Ser Lys Lys Pro Lys Ser
    50                  55                  60

Val Thr Asp Ile Tyr Leu Leu Asn Leu Ala Leu Ser Asp Leu Leu Phe
65                  70                  75                  80

Val Ala Thr Leu Pro Phe Trp Thr His Tyr Leu Ile Asn Glu Lys Gly
                85                  90                  95

Leu His Asn Ala Met Cys Lys Phe Thr Thr Ala Phe Phe Phe Ile Gly
            100                 105                 110

Phe Phe Gly Ser Ile Phe Phe Ile Thr Val Ile Ser Ile Asp Arg Tyr
        115                 120                 125

Leu Ala Ile Val Leu Ala Ala Asn Ser Met Asn Asn Arg Thr Val Gln
    130                 135                 140

His Gly Val Thr Ile Ser Leu Gly Val Trp Ala Ala Ala Ile Leu Val
145                 150                 155                 160

Ala Ala Pro Gln Phe Met Phe Thr Lys Gln Lys Glu Asn Glu Cys Leu
                165                 170                 175

Gly Asp Tyr Pro Glu Val Leu Gln Glu Ile Trp Pro Val Leu Arg Asn
            180                 185                 190

Val Glu Thr Asn Phe Leu Gly Phe Leu Leu Pro Leu Leu Ile Met Ser
        195                 200                 205

Tyr Cys Tyr Phe Arg Ile Ile Gln Thr Leu Phe Ser Cys Lys Asn His
    210                 215                 220

Lys Lys Ala Lys Ala Ile Lys Leu Ile Leu Leu Val Val Ile Val Phe
225                 230                 235                 240

Phe Leu Phe Trp Thr Pro Tyr Asn Val Met Ile Phe Leu Glu Thr Leu
                245                 250                 255

Lys Leu Tyr Asp Phe Phe Pro Ser Cys Asp Met Arg Lys Asp Leu Arg
            260                 265                 270
```

Leu Ala Leu Ser Val Thr Glu Thr Val Ala Phe Ser His Cys Cys Leu
        275                     280                     285

Asn Pro Leu Ile Tyr Ala Phe Ala Gly Glu Lys Phe Arg Arg Tyr Leu
    290                     295                     300

Tyr His Leu Tyr Gly Lys Cys Leu Ala Val Leu Cys Gly Arg Ser Val
305                     310                     315                     320

His Val Asp Phe Ser Ser Ser Glu Ser Gln Arg Ser Arg His Gly Ser
                325                     330                     335

Val Leu Ser Ser Asn Phe Thr Tyr His Thr Ser Asp Gly Asp Ala Leu
            340                     345                     350

Leu Leu Leu
        355

<210> 3
<211> 2300
<212> DNA
<213> Homo sapiens

<400> 3
agatgctcag ccgcagacct ttgggagagt aaagggggca cacccaccca ccttgcctcc 60

aggctctttc cttcctattc ctgttctatg gtggggctcc attgcgagac ttcagattga 120

gaaatcagat gaagtttcaa gaaaaggaaa ctggcaggtg acagagatgg gtggagggac 180

tggggaaagg ctgtttactc cctcctgtct agtcggcttg gtccctttag ggctccggat 240

atctttggtg acttgtccac tccagtgtgg catcatgtgg cagctgctcc tcccaactgc 300

tctgctactt ctagtttcag ctggcatgcg gactgaagat ctcccaaagg ctgtggtgtt 360

cctggagcct caatggtaca gcgtgcttga gaaggacagt gtgactctga agtgccaggg 420

agcctactcc cctgaggaca attccacaca gtggtttcac aatgagaacc tcatctcaag 480

ccaggcctcg agctacttca ttgacgctgc cacagtcaac gacagtggag agtacaggtg 540

ccagacaaac ctctccaccc tcagtgaccc ggtgcagcta gaagtccata tcggctggct 600

gttgctccag gcccctcggt gggtgttcaa ggaggaagac cctattcacc tgaggtgtca 660

cagctggaag aacactgctc tgcataaggt cacatattta cagaatggca aagacaggaa 720

gtattttcat cataattctg acttccacat tccaaaagcc acactcaaag atagcggctc 780

ctacttctgc agggggcttg ttgggagtaa aaatgtgtct tcagagactg tgaacatcac 840

catcactcaa ggtttggcag tgtcaaccat ctcatcattc tctccacctg ggtaccaagt 900

ctctttctgc ttggtgatgg tactcctttt tgcagtggac acaggactat atttctctgt 960

gaagacaaac atttgaagct caacaagaga ctggaaggac cataaactta aatggagaaa 1020

ggaccctcaa gacaaatgac cccatcccca tgggagtaat aagagcagtg gcagcagcat 1080

ctctgaacat ttctctggat ttgcaacccc atcatcctca ggcctctcta caagcagcag 1140

```
gaaacataga actcagagcc agatccttta tccaactctc gatttttcct tggtctccag     1200
tggaagggaa aagcccatga tcttcaagca gggaagcccc agtgagtagc tgcattccta     1260
gaaattgaag tttcagagct acacaaacac tttttctgtc ccaaccattc cctcacagca     1320
aagcaacaat acaggctagg gatggtaatc ctttaaacat acaaaaattg ctcgtattat     1380
aaattaccca gtttagaggg aaaaaaagaa aataattatt cctaaacaaa tggataagta     1440
gaattaatgg ttgaggcagg accctacaga gtgtgggaac tgctggggat ctagagaatt     1500
cagtgggacc aatgaaagca tggctgagaa atagcagggt agtccaggat agtctaaggg     1560
aggtgttccc atctgagccc agagataagg gtgtcttcct agaacattag ccgtagtgga     1620
attaacagga aatcatgagg gtgacgtaga attgagtctt ccaggggact ctatcagaac     1680
tggaccattt ccaagtatat aacgatgagc cctctaatgc taggagtagc aaatggtcct     1740
aggaagggga ctgaggattg gggtgggggt ggggtggaaa agaaagtaca gaacaaaccc     1800
tgtgtcactg tcccaagtta agctaagtga acagaactat ctcagcatca gaatgagaaa     1860
gcctgagaag aaagaaccaa ccacaagcac acaggaagga aagcgcagga ggtgaaaatg     1920
ctttcttggc cagggtagta agaattagag gttaatgcag ggactgtaaa accacctttt     1980
ctgcttcaat gtctagttcc tgtatagctt tgttcattgc atttattaaa caaatgttgt     2040
ataaccaata ctaaatgtac tactgagctt cactgagtta cgctgtgaaa ctttcaaatc     2100
cttcttcatg tcagttccaa tgaggtgggg atggagaaga caattgttgc ttatgaaaga     2160
aagctttagc tgtctctgtt ttgtaagctt tcagtgcaac atttcttggt tccaataaag     2220
cattttacaa gatcttgcat gctactctta gatagaagat ggcaaaacca tggtaataaa     2280
atatgaatga taaaaaaaaa                                                 2300

<210>  4
<211>  233
<212>  PRT
<213>  Homo sapiens

<400>  4

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Ser Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Asn Leu Ile Ser Ser Gln Ala Ser Ser Tyr Phe Ile Asp Ala Ala Thr
65                  70                  75                  80
```

Val Asn Asp Ser Gly Glu Tyr Arg Cys Gln Thr Asn Leu Ser Thr Leu
85 90 95

Ser Asp Pro Val Gln Leu Glu Val His Ile Gly Trp Leu Leu Leu Gln
100 105 110

Ala Pro Arg Trp Val Phe Lys Glu Glu Asp Pro Ile His Leu Arg Cys
115 120 125

His Ser Trp Lys Asn Thr Ala Leu His Lys Val Thr Tyr Leu Gln Asn
130 135 140

Gly Lys Asp Arg Lys Tyr Phe His His Asn Ser Asp Phe His Ile Pro
145 150 155 160

Lys Ala Thr Leu Lys Asp Ser Gly Ser Tyr Phe Cys Arg Gly Leu Val
165 170 175

Gly Ser Lys Asn Val Ser Ser Glu Thr Val Asn Ile Thr Ile Thr Gln
180 185 190

Gly Leu Ala Val Ser Thr Ile Ser Ser Phe Ser Pro Pro Gly Tyr Gln
195 200 205

Val Ser Phe Cys Leu Val Met Val Leu Leu Phe Ala Val Asp Thr Gly
210 215 220

Leu Tyr Phe Ser Val Lys Thr Asn Ile
225 230

<210> 5
<211> 702
<212> DNA
<213> Homo sapiens

<400> 5
atgtggcagc tgctcctccc aactgctctg ctacttctag tttcagctgg catgcggact 60

gaagatctcc caaaggctgt ggtgttcctg gagcctcaat ggtacagcgt gcttgagaag 120

gacagtgtga ctctgaagtg ccagggagcc tactcccctg aggacaattc cacacagtgg 180

tttcacaatg agaacctcat ctcaagccag gcctcgagct acttcattga cgctgccaca 240

gtcaacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaaga caggaagtat tttcatcata attctgactt ccacattcca 480

aaagccacac tcaaagatag cggctcctac ttctgcaggg ggcttgttgg gagtaaaaat 540

gtgtcttcag agactgtgaa catcaccatc actcaaggtt tggcagtgtc aaccatctca 600

tcattctctc cacctgggta ccaagtctct ttctgcttgg tgatggtact cctttttgca 660

gtggacacag gactatattt ctctgtgaag acaaacattt ga 702

<210> 6
<211> 233
<212> PRT
<213> Homo sapiens

<400> 6

Met Trp Gln Leu Leu Leu Pro Thr Ala Leu Leu Leu Leu Val Ser Ala
1 5 10 15

Gly Met Arg Thr Glu Asp Leu Pro Lys Ala Val Val Phe Leu Glu Pro
20 25 30

Gln Trp Tyr Ser Val Leu Glu Lys Asp Ser Val Thr Leu Lys Cys Gln
35 40 45

Gly Ala Tyr Ser Pro Glu Asp Asn Ser Thr Gln Trp Phe His Asn Glu
50 55 60

Asn Leu Ile Ser Ser Gln Ala Ser Ser Tyr Phe Ile Asp Ala Ala Thr
65 70 75 80

Val Asn Asp Ser Gly Glu Tyr Arg Cys Gln Thr Asn Leu Ser Thr Leu
85 90 95

Ser Asp Pro Val Gln Leu Glu Val His Ile Gly Trp Leu Leu Leu Gln
100 105 110

Ala Pro Arg Trp Val Phe Lys Glu Glu Asp Pro Ile His Leu Arg Cys
115 120 125

His Ser Trp Lys Asn Thr Ala Leu His Lys Val Thr Tyr Leu Gln Asn
130 135 140

Gly Lys Asp Arg Lys Tyr Phe His His Asn Ser Asp Phe His Ile Pro
145 150 155 160

Lys Ala Thr Leu Lys Asp Ser Gly Ser Tyr Phe Cys Arg Gly Leu Val
165 170 175

Gly Ser Lys Asn Val Ser Ser Glu Thr Val Asn Ile Thr Ile Thr Gln
180 185 190

Gly Leu Ala Val Ser Thr Ile Ser Ser Phe Ser Pro Pro Gly Tyr Gln
195 200 205

Val Ser Phe Cys Leu Val Met Val Leu Leu Phe Ala Val Asp Thr Gly
210 215 220

Leu Tyr Phe Ser Val Lys Thr Asn Ile
225 230

<210> 7
<211> 21
<212> DNA
<213> Artificial sequence

<220>
<223> PRIMER CX3CR1 S

<400> 7
ccgaggtcct ccaggaaatc t 21

<210> 8
<211> 22
<212> DNA
<213> Artificial sequence

<220>
<223> PRIMER CX3CR1 R

<400> 8
tcagcatcag gttcaggaac tc 22

<210> 9
<211> 20
<212> DNA
<213> Artificial sequence

<220>
<223> PRIMER FCRN S

<400> 9
agtcaaaagt ggcgatgagc 20

<210> 10
<211> 20
<212> DNA
<213> Artificial sequence

<220>
<223> PRIMER FCRN R

<400> 10
ctgccgtgag tagcaagaca 20

<210> 11
<211> 19
<212> DNA
<213> Artificial sequence

<220>
<223> PRIMER FCGR2A S

<400> 11
ccaggaggga gaaaccatc 19

<210> 12
<211> 20
<212> DNA
<213> Artificial sequence

<220>

<223> PRIMER FCGR2A R

<400> 12
ctcttctccc ctccctacat 20

<210> 13
<211> 21
<212> DNA
<213> Artificial sequence

<220>
<223> PRIMER FCGR3A S

<400> 13
atatttacag aatggcacag g 21

<210> 14
<211> 32
<212> DNA
<213> Artificial sequence

<220>
<223> PRIMER FCGR3A R

<400> 14
acgtgctgag cttgagtgat ggtgatgttc ac 32

<210> 15
<211> 20
<212> DNA
<213> Artificial sequence

<220>
<223> PRIMER FCGR3A S

<400> 15
tcctaatagg tttggcagtg 20

<210> 16
<211> 20
<212> DNA
<213> Artificial sequence

<220>
<223> PRIMER FCGR3A R

<400> 16
aaatgttcag agatgctgct 20

<210> 17
<211> 19
<212> DNA
<213> Artificial sequence

<220>
<223> PRIMER FCGR3B S

<400> 17
aaaggctgtg gtgttcctg 19

<210> 18
<211> 22

<212> DNA
<213> Artificial sequence

<220>
<223> PRIMER FCGR3B R

<400> 18
cctttgtttc accctttatt gg 22

<210> 19
<211> 20
<212> DNA
<213> Artificial sequence

<220>
<223> Primer CX3CR1 (249)

<400> 19
tcttctggac accctacaac 20

<210> 20
<211> 20
<212> DNA
<213> Artificial sequence

<220>
<223> Primer CX3CR1 (280)

<400> 20
ggccctcagt gtgactgaga 20

<210> 21
<211> 20
<212> DNA
<213> Artificial sequence

<220>
<223> Primer FCRN (287)

<400> 21
ggggctggcg cagcccctca 20

<210> 22
<211> 20
<212> DNA
<213> Artificial sequence

<220>
<223> Primer FCRN (298)

<400> 22
aatctccagc caagtcctcc 20

<210> 23
<211> 20
<212> DNA
<213> Artificial sequence

<220>
<223> Primer FcgR2A (139)

<400> 23
tccaggaggg agaaaccatc 20

<210> 24
<211> 20
<212> DNA
<213> Artificial sequence

<220>
<223> Primer FcgR2A (166)

<400> 24
aaaatcccag aaattctccc 20

<210> 25
<211> 20
<212> DNA
<213> Artificial sequence

<220>
<223> Primer FcgR2A (204)

<400> 25
aagcctgtga ccatcactgt 20

<210> 26
<211> 20
<212> DNA
<213> Artificial sequence

<220>
<223> Primer FcgR3A (147)

<400> 26
atatttacag aatggcaaag 20

<210> 27
<211> 20
<212> DNA
<213> Artificial sequence

<220>
<223> Primer FcgR3A (158)

<400> 27
ttcatcataa ttctgacttc 20

<210> 28
<211> 20
<212> DNA
<213> Artificial sequence

<220>
<223> Primer FcgR3A (166)

<400> 28
ccaaaagcca cactcaaaga 20

<210> 29
<211> 20
<212> DNA
<213> Artificial sequence

<220>

<223> Primer FcgR3A( 176)

<400> 29
cctacttctg cagggggctt 20

<210> 30
<211> 19
<212> DNA
<213> Artificial sequence

<220>
<223> Primer FcgR3A (203)

<400> 30
tcaaccatct catcattct 19

<210> 31
<211> 20
<212> DNA
<213> Artificial sequence

<220>
<223> Primer FcgR3A (234)

<400> 31
tctctgtgaa gacaaacatt 20

<210> 32
<211> 20
<212> DNA
<213> Artificial sequence

<220>
<223> Primer FcgR3A (245)

<400> 32
gagactggaa ggaccataaa 20

<210> 33
<211> 20
<212> DNA
<213> Artificial sequence

<220>
<223> Primer FcgR3B (65)

<400> 33
aggcctggct tgagatgagg 20

<210> 34
<211> 20
<212> DNA
<213> Artificial sequence

<220>
<223> Primer FcgR3B (106)

<400> 34
cggtgcagct agaagtccat 20

<210> 35
<211> 20

<212> DNA
<213> Artificial sequence

<220>
<223> Primer CD16AB-328F

<400> 35
ccggtgcagc tagaagtcca 20

<210> 36
<211> 21
<212> DNA
<213> Artificial sequence

<220>
<223> Primer CD16AB-522R

<400> 36
ttccagctgt gacacctcag g 21

<210> 37
<211> 21
<212> DNA
<213> Artificial sequence

<220>
<223> Primer CD16 F

<400> 37
ccggtgcagc tagaagtcca t 21

<210> 38
<211> 27
<212> DNA
<213> Artificial sequence

<220>
<223> Primer CD16 158F R

<400> 38
ctctgaagac acatttttac tcccaaa 27

<210> 39
<211> 27
<212> DNA
<213> Artificial sequence

<220>
<223> Primer CD16 158V R

<400> 39
ctctgaagac acatttttac tcccaac 27

<210> 40
<211> 21
<212> DNA
<213> Artificial sequence

<220>
<223> Primer CX3CR1-35 F

<400> 40
ggctgactgg cagatccaga g 21

```
<210>  41
<211>  22
<212>  DNA
<213>  Artificial sequence

<220>
<223>  Primer CX3CR1-262 R

<400>  41
tgacactctt gggcttcttg ct                                              22
```

## Description

### Field of the invention

The present invention relates to the domain of immune cell activation and more specifically to an in vitro method to evaluate host cellular humoral activity, and the associated kit testing

### Background of the Invention

Innate NK cell activation towards a target can be immediately induced providing that NK cell receptors mediated recognition of inhibitory and activating ligand on the target cell favors induction of cytotoxic potential or production of inflammatory cytokines such as IFN-g.

Target cell inhibitory ligand essentially consist of constitutively expressed MHC class I molecules ( HLA-A, B, C , G, E) that are recognized by a highly diversified NK cell repertoire of receptors thus preventing auto reactivity of NK cells toward most cells of the organisms bearing HLA class I, except for immunopriviledge sites such as neurones or trophobast cells. Stress or pathogen induced activation antigens, such as ligands for NKG2D, NCR, CX3CR1 or DNAM receptors (such as MICA/B; ULBP, fractalkine, CD155 or CD112 ..) can be up regulated at the surface of immune effector cells during inflammatory, viral or neoplasic challenge associated to many diseases. These ligands have been identified as major target signals that modulate both T cell and NK cell immune activation during neoplasic, infectious, autoimmune, inflammatory diseases, pregnancy and alloimmune disorders. As numerous studies suggested their critical role in diseases, therapeutic approaches to either block or to enhance the interactions between immune NK receptors and their cognate ligands may have important implications for human health.

In particular, the role of the fractalkine-CX3CP1 pathway has been involved in various clinical settings, identifying the CX3CR1-fractalkine axis as a therapeutic target to attenuate pain and neuropathogenic processes, inflammatory renal and cardiovascular disorders, auto immune diseases, HIV infection progression, colorectal, pancreatic, and hepatocellular cancer (recently reviewed in D'HAESE et al., Expert opinion on therapeutic targets., vol.14, p:207-219, 2010). The G-to-A transition changing Val249 to Iso (V249I) and a C-to-T transition changing Thr280 to Met (T280M) were shown to be common in the population. Patients homozygous for the CX3CR1 mutation were shown to have increased susceptibility to HIV infection and a more rapid progression to AIDS. Genetic polymorphism of the CX3CR1 receptor confers differential immune activation by fractalkine mediated M280/I289 variants have been associated with reduction of endothelial dysfunction, artheroprotective effect in coronary artery diseases and KO of the CX3CR1 KO was associated to enhanced graft survival in animal models. (DAMAS et al., Arteriosclerosis, thrombosis, and vascular biology., vol.25, p:2567-2572, 2005). A trend towards higher soluble fractalkine levels is observed in many diseases in association with endothelial dysfunction (review in UMEHARA et al., Arteriosclerosis, thrombosis, and vascular biology, vol.24, p:34-40, 2004) such as neurological diseases, vasculopathy (BJERKELI et al., Rheumatology, vol.46, p:1422-1427, 2007), and preeclampsia (STEPANIAN et al., PloS one, vol.4, e6192, 2009). Fractalkine-dependent cell-cell adhesion under conditions of physiologic shear was severely reduced in cells expressing CX3CR1-M280, and this was associated with marked reduction in the kinetics of fractalkine binding as well as reduced fractalkine-induced chemotaxis of primary leukocytes from donors homozygous for CX3CR1-M280. CX3CR1-M280 was independently associated with a lower risk of cardiovascular disease (adjusted odds ratio, 0.60; $p = 0.008$).

Apart from their modulation consequent to pathogen associated ligand, NK cells are also unique innate immune effector cells that respond to antibodies. Efficiency of humoral responses mediated by antibody recognition of their target depends both on activation of the complement cascade and on antibody dependent cell cytotoxicity (ADCC), both mediated by Fc fragment of IgG antibodies, and in particular activation can be achieved after engagement of the CD16 NK cell Fc receptor. Providing their ability to recognize their target specific antigen, the Fc fragment of IgG antibodies provide powerfull activating signals to innate immune effector cells expressing Fc Receptor (FcR), such as CD16 but as we show also affect CX3CR1 expression. Considering that polymorphism of FCR (in particular CD32 and CD16) and CX3CR1 receptors affects their capacity to sense their respective IgFc and fractalkine ligands, combined analysis of CD16 and CX3CR1 expression profiles gives information on a subject's individual capacity to mount humoral and natural immune responses after allogenic and pathological challenges.

In particular, we show that in allogeneic transplantation situations presence of anti HLA antibodies can bypass inhibitory MHC class I signalling of natural NK cytotoxicity and is a very potent mechanism to mediate injury of target cells recognized by antibodies after eliciting FcR NK mediated ADCC and CX3CR1 signalling.

Thus, various alloimmune (pregnancy, transplantation, transfusion, ...), and autoimmune pathogenic challenges can orient ADCC and natural immune innate cell activation potential of a host. Combination of genetic variability conferred by CX3CR1 and CD16 polymorphisms further enhance the heterogeneity of a given individual to mount an immune response when triggered by humoral or fractalkine signalling. Non invasive methods that evaluate the individual profile of a subject with regards to humoral CD16 and Fractalkine-CX3CR1 regulated pathways may thus improve both monitoring and prognosis in various clinical settings but also provide new tools to assay efficiency of novel therapeutic strategies targeting these FcR and CX3CR1 pathways. Depending upon the situation, development of strategies that either block or enhance the interactions between FcR, CX3CR1 and their ligands may have important implications for human health and disease. Monitoring tools that allow evaluation of such strategies thus represent a major challenge to achieve, notably when relevant development implicate Fc engineered compound interfering with immune ADCC activation. As the fractalkine/CX3CR1 axis bears a major potential in the design of therapeutic approaches in various clinical diseases, the present demonstration linking humoral cellular reaction to CX3CR1 signalling may bring clues to the better understanding of manipulation of the CX3CR1 dependent response. Indeed most of these strategies involve use of CX3CR1 neutralizing or blocking or fractalkine antagonists. In this perspective, the potential of therapeutic Fc antibodies to modulate CX3CR1 should have various implications on immune pathways that may orient better translation of CX3CR1 as a therapeutic target in clinical settings such as inflammatory, Central Nervous System , neoplastic , cardiovascular, pancreatic and alloimmune disorders.

It is well known that antibodies reactive with auto or alloantigens, which expression are induced on endothelial cells or antigen presenting cells during a pathogenic process have a deleterious effect in several settings of human auto immune diseases, organ transplantations, and pregnancy diseases. Still, although routine seric detection of the auto and alloimmune antibody specificities exquisitely refines diagnosis by various improvement in the sensibility of ELISA or luminex assay of anti HLA alloantibodies, anti phospholipids , ANCA, monitoring tools evaluating the functional pathogenic effect of these antibodies or host immune efficiency to mediate humoral injury remains limited.

In particular allogenic or autoimmune antigens expressed on endothelial cells are thought to be major major targets of humoral responses that control graft rejection and cardiovascular disorders in transplantation settings. We also show that NK mediated antibody cellular effector function can be directed against progenitor endothelial cells and thus impair host potential to repair these lesions. These humoral response may thus represent a major limit to the devellopement of cellular therapy using the repair potential of progenitor cells in regenerative medecine, notably if allogeneic progenitor cells such as cord blood derived cells are intented for treatment.

Presence of donor lymphocyte-reactive Human Leukocyte Antigen (HLA)-specific antibodies either before and/or after transplantation has been associated with hyperacute rejections, early acute rejections, and long term loss of graft function (chronic rejection). However, rejections may occur in the absence of detectable anti HLA antibodies, suggesting that non-HLA antigenic systems may also play a role in graft or foetal loss. Expression of the major histocompatibility class I-related chain A antigen (MICA a ligand of the NKG2D receptor) on endothelial cells was identified as one of the target antigens of humoral immunity associated with irreversible endothelial lesions and rejections of kidney allografts. Studies of HLA-identical living-related donor allografts showed that non HLA endothelial cell/APC reactive antibodies correlated with rejection, graft loss, and poor allograft function. Deleterious activity of antibodies towards the graft can thus occur through antibody recognition of alloantigen or stress/pathogen induced autoantigen on graft cells and could be responsible for up to 80% of irreversible rejections in patients grafted in HLA matched settings.

When appropriate, various tests of transplant recipient serum are in use to anticipate these pathogenic effects of antibodies toward the graft, and orient matching of donor-recipient compatibility in kidney transplant. The assay of complement dependent cytotoxicity (CDC) remains poorly sensitive, and only reveals complement dependant lytic humoral activity. It was for years the main technique for the final selection of the transplant (cross-match) since it estimates the cell lysis of the donor in the presence of serum of the recipient, but this technique does not assay the capacity of the recipient to mediate a cellular antibody mediated immune response. The luminex test detects presence of HLA antibodies in the host serum that may be directed against the transplant in a very sensitive way, but this technique is now criticized because it only allows anticipating one third of acute humoral rejection and is restricted to detection of anti HLA and more recently anti -MICA antibodies. Furthermore, these techniques evaluate the complement dependent or not component of the serum with regard to humoral activation against donor targets but do not account for the host immune system individual variability in capacity to mount an efficient cellular immune response, which can notably be genetically determined by polymorphism and expression profile of at least FcR, CX3CR1 and NKG2D immune receptors of a subject.

Thus, although very sensitive Luminex techniques are available to detect the presence of alloantibodies in recipient serum, these are limited to detection of anti HLA antibodies reactivity of the serum and extrapolation of their deleterious effect on HLA alleles expressed on graft cells. In this setting of the background of the invention, there are currently no suitable method to assay the functional and pathogenic effect of antibodies against their target antigen in a routine target-specific cell-crossmatch, taking into account a given host immune cell mediated reactive potential.

## Summary of the invention

In a first aspect, the present invention provides an *in vitro* method for determining a subject cellular humoral activity, which comprises the step of:

(ii) determining the expression profile of at least the CX3CR1 gene in effector cells expressing FCRG and CX3CR1 , preferably $CD3^-CD56^+$ NK cells; and

(iii) comparing said expression profile to at least one control such as control values of FCRG and CX3CR1 expressing subsets in representative healthy donor control cohorts

In a preferred embodiment, said method is for determining a subject cellular humoral activity directed against a specific antigen which comprises the step of:

(i) incubating :

- effector cells corresponding to FCRG and CX3CR1 expressing cells, preferably NK cells;
- Serum, or any medium containing therapeutic Fc compound or compounds interfering with the fractalkine CX3CR1 pathways, preferably a serum from said subject; and
- a target antigen preferentially expressed at the surface of a cell;

(ii) determining the expression profile of at least the CX3CR1 in said effector cells, preferably by flow cytometry analysis; and

(iii) comparing said expression profile to at least one control.

In a second aspect, the present invention refers to a kit for determining a subject cellular humoral activity comprising (i) at least one mean for determining the expression profile of CX3CR1 gene in effector cells expressing FCRG and CX3CR1, preferably in lymphocytes, $CD3^-CD56^+$ NK cells and monocytes.

Finally, the present invention refers to the use, for determining a subject cellular humoral activity directed against an antigen, of the above mentioned kit.

## Detailed description of the invention

The present invention is based on the discovery by the present inventors of the existing link between humoral immunity (i.e. presence of reactive antibodies) and CX3CR1 and FcR receptor dependant activation of immune NK cell effector function.

More specifically, the inventors have demonstrated that natural anti-HLA Alloantibodies, anti endothelial antibodies present in the serum of immunized transplanted patients or women experiencing reproductive implantation failure or human Fc therapeutic antibodies have the capacicity to induce activation of host dependant innate immune effector cell function towards allogenic target cells expressing the membrane antigen specificities recognized by the antibodies (i.e. B lymphocytes and endothelial cells or endothelial precursor cells). The invention provides a link between humoral FcR mediated NK activation (NK cell degranulation, target cell lysis, Interferon production,) and serum or Fc humanized antibody induced modulation of NK receptors (preferably CX3CR1 and CD16) and target cell microparticle release that allow evaluation of humoral cellular activation of a given host potential to mount cellular immune responses. This result provide a method to assay the potentially pathogenic activity of an antibody mediated by ADCC (i.e. Antibody-Dependant Cell Cytotoxicity) of a subject and to index the subject's immune potential to induce antibody mediated target cell injury.

Particularly, the inventors have also demonstrated that antibody mediated NK cell activation resulting in a decrease in CD16 cells' surface expression is correlated to a decrease of CX3CR1 expression in blood $CD3^-CD56^+$ NK cells of patients. The intensity of the antibody dependent cell cytototoxicity (ADCC) is dependant of combination of the host expression of these receptors at the surface of immune effector cells. More specifically, the inventors show that specific antibodies induced activation of $CD3^-CD56^+$ NK cells towards cell targets recognized by antibodies associated to down regulation of NK cells' surface CX3CR1 and CD16 expression and release of target cell derived microparticles.

Thus, in a first aspect, the present invention provides an *in vitro* method for determining a subject cellular humoral activity, which comprises the step of:

(ii) determining the expression profile of at least the CX3CR1 gene in effector cells expressing FCRG and CX3CR1 , preferably $CD3^-CD56^+$ NK cells

(iii) comparing said expression profile to at least one control such as control values of FCRG and CX3CR1 expressing subsets in representative healthy donor control cohorts.

In a preferred embodiment, said method is for determining a subject cellular humoral activity directed against a specific antigen which comprises the step of:

(i) incubating :

- effector cells corresponding to FCRG and CX3CR1 expressing cells, preferably to NK cells (as used herein NK cells can refer to NK cells present in whole blood cells, in isolated peripheral mononuclear blood cells, NK cell lines, and to NK cells transfected with CX3CR1 and eventually CD16 variants),
- Serum, or any medium containing therapeutic Fc compound or compounds interfering with the fractalkine CX3CR1 pathways, preferably a serum from said subject; and
- a target antigen preferentially expressed at the surface of a cell;

(ii) determining the expression profile of at least the CX3CR1 gene in said effector cells, preferably by flow cytometry analysis; and

(iii) comparing said expression profile to at least one control.

As used herein, the term "subject" refers to a mammal, preferably a human.

As used herein "CX3CR1 gene" refers to chemokine (C-X3-C motif) receptor 1 (GeneID: 1524) gene having the nucleic acid sequence SEQ ID N°1 coding for the polypeptide having the amino acid sequence SEQ ID N°2.

As used herein, the term "subject cellular humoral activity directed against an antigen" refers to the existence in said subject of antibodies reacting with said antigen and inducing an ADCC mechanism against cells bearing said antigen.

Said antibodies are alloantibodies when they react with alloantigens produced by individuals of the same species. Said antibodies can also be autoantibodies produced by the same subject when they react with a self constitutively expressed autoantigen or an autoantigen induced by a pathogenic process.

As used herein, the term "NK cells" refers to $CD3^-CD56^+$ NK cells, preferably to $CD3^-CD56^+CD16^+$ NK cell subsets.

Preferably, said target antigen is an alloantigen or an autoantigen.

According to another preferred embodiment, the method of the invention comprises the step (ii) of determining the expression profile of at least the CX3CR1 gene and of at least one FCRG gene, preferentially said FCRG gene is CD16 gene.

As used herein "FCRG gene" corresponds to Fc gamma receptors genes, which bind to the Fc portion of IgG antibodies.

As used herein "CD16 gene" refers to FCGR3A (GeneID 2214) and FCGR3B (Gene ID 2215). FCGR3A gene has the nucleic acid sequence SEQ ID N°3 coding for the polypeptide having the amino acid sequence SEQ ID N°4. FCGR3B gene has the nucleic acid sequence SEQ ID N°5 coding for the polypeptide having the amino acid sequence SEQ ID N°6.

As used herein, the "expression profile" refers to the level of transcript or protein expression and/or to the genetic polymorphism of CX3CR1 and eventually of CD16 genes, preferably refers to the protein expression level of CX3CR1 and eventually of CD16 genes, and most preferably to the cell surface protein expression level of CX3CR1 and eventually of CD16 genes.

Methods for determining the expression level of a gene are well known for the man skilled in the art.

This determining step can be assessed by analyzing the expression of mRNA transcript or mRNA precursors, such as nascent RNA, of said gene or by analyzing the expression of the protein translated from said gene, preferably by flow cytometry analyzis of the cell surface expression of the protein translated from said gene.

The expression of mRNA transcript or mRNA precursors can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TAQMAN, SYBERGREEN), and probes arrays such as GENECHIP™ DNA Arrays (AFFYMETRIX). Advantageously, the analysis of the expression level of mRNA transcribed from said genes involves the process of nucleic acid amplification, e. g., by RT-PCR (the experimental embodiment set forth in U. S. Patent No. 4,683, 202), ligase chain reaction (BARANY, Proc. Natl. Acad sci USA, vol. 88, p: 189-193, 1991), self sustained sequence replication (GUATELLI et al., Proc. Natl Acad Sci. USA, vol.87, p: 1874-1878, 1990), transcriptional amplification system (KWOH et al., 1989, Proc. Natl Acad Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

As described in the examples, a protocol for determining the genetic polymorphism of CX3CR1 and CD16 genes or to detect levels of CD16 and CX3CR1 transcripts using quantitative Q-PCR is described but any primer pair may be designed to distinguish transcript or genomic variants of FCR and CX3CR1 that may relate to cognate Fc or fractalkine ligand binding efficiency to mediate NK cell activation .

The analysis of the expression of the protein(s) translated from said gene(s) can be assessed using an antibody *(e.g.,* a radio-labeled, chromophore-labeled, fluorophore-labeled, or enzyme-labeled antibody), an antibody derivative *(e.g.,* an antibody conjugate with a substrate or with the protein or ligand of a protein of a protein/ligand pair *(e.g.,* biotin-streptavidin), or an antibody fragment *(e.g.,* a single-chain antibody, an isolated antibody hypervariable domain, *etc.)* an ingeniered or recombinant protein interfering with the CX3CR1 pathway such as fractalkine/CX3CR1 agonist or antagonist, which may interfere with specific binding to the protein translated from CX3CR1 . Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA), Flow cytometry (FACS).

Antibodies that can be used for these analysis are well known from the skilled person and include, as an example, the Anti-Human CX3CR1 Antibodies, PE or FITC conjugated commercialized by MBL INTERNATIONAL OR BIOLEGEND for Flow Cytometry, the Rabbit Anti-CX3CR1 / V28 (NT) Polyclonal Antibody, Unconjugated commercialised BYPROSCI, INC for Flow Cytometry, the mouse anti-Human CX3CR1 Monoclonal Antibody, Unconjugated, Clone 2B11 commercialized by ABNOVA CORPORATION for ELISA, the anti CD16 3G8 conjugated antibody commercialized by BECKMAN COULTER, the mouse AntiCD16 Monoclonal Antibody, Allophycocyanin Conjugated, Clone LNK16 commercialzed by ABCAM for Flow cytometry, or the mouse Anti-Human CD16 (HI16a) Monoclonal Antibody FITC Conjugated commercialized by ABBIOTEC for ELISA and Flow Cytometry.

Detection of antibody binding to target antigen can be revealed in serum after incubating relevant cells (cells prepared from tissues, blood, lymphocytes, cell lines or endothelial primary cultures) with 10-20% of serum or various concentrations of therapeutic IgG or Fc bearing coumpound ( IVIG, ATG antithymoglobulines, any humanized or Fc bearing immunosuppressive or therapeutic antibodies, anti endothelial antibodies, NKG2D or recombinant protein of interest fused to the human fc fragment...) and revelation of antibody staining using anti human Fc Fab'2 PE or FITC labelled secondary antibodies or alexa fluor labelled protein G or protein A human Fc binding reagent.

In a still preferred embodiment, the method of the invention further comprises the step of determining the lysis of the cell expressing said target antigen at its surface.

Said cells lysis can be determined by well known methods such as by flow cytometry detection of cell death and survival using any adapted cell trackers (CFSE, DID, CMFDA, Chromium 51, ....) combined with conjugated antibody staining of cells for annexin, 7AAD and/ or cell specific markers (of cells expressing target antigen) or in a preferred method by identifying the origin of death associated bodies resulting from cell lysis though enumeration of Microvesicules <1 microM labelled by a cell tracker and gated both on size criteria (< 3 microM can be further gated on 1-3 and 0.5-1 microM death associated microvesicules, the latter being referred to as microparticles) .

Preferably, said cell lysis is determined by flow cytometry is assessed through analysis of the percentage of Annexin 7AAD double labeled cells traducing cell death, quantification of cell death bodies (1-3micrometers in size), and/or by quantification of microparticles ($<1\mu M$) resulting from incubation of the cell expressing the target antigen at its surface with effector cells in absence or in presence of serum.

Most preferably, said cells lysis is determined by quantification of microparticles ($<1\mu M$).

According to a forth preferred embodiment, the expression profile-determining step (ii) of the method of the invention is assessed by analysing the expression profile of the protein(s) translated from said CX3CR1 and eventually CD16 gene(s).

Advantageously, the expression profile-determining step (ii) of the method of the invention is assessed by analysing the cell surface expression profile of the protein(s) translated from said gene(s).

Preferably, this expression profile-determining step is assessed by Flow cytometry.

As used herein, a "control" corresponds to the expression profile of at least CX3CR1 and eventually CD16 gene(s) in NK cells incubated alone, in NK cells incubated with cells expressing target antigen without serum, and/or in NK cells incubated with serum and without cells expressing target antigen.

Preferably, the method further comprises the step (v) of determining whether the expression level (which expression level can correspond to the % and Mean Fluorescent Intensity (Mfi) of positive cells in flow cytometry) of said CX3CR1 and eventually CD16 gene(s) is(are) increased or decreased relative to the control, preferably said control correspond to target antigen and NK cells without serum; wherein a decreased expression level of said gene(s) is indicative of an increased cellular humoral activity directed against said antigen of said subject.

In a preferred method, the difference between 1/ %CX3CR1 [(Target + FcC+ effector cells)- (Target + effector cells) ] and 2/ CD16mfi [(Target +FcC+ effector cells)- (target + effector cells) ] observed in absence or presence or Fc compound (serum antibodies or therapeutic IgG or humanized therapeutic Fc fusion protein or antibodies) is evaluated. In a preferred method the Control values observed for % and Mfi of CD16, CX3CR1 and CD107a/LAMP-1/lamp activation markers are set to 100% and relative values represent the variation in % observed in reference to controls after introduction of serum or therapeutic compound.

More especially, the inventors have established that a decrease in CX3CR1 and eventually in CD16 mfi within CD3-CD56+ NK cells' expression is associated with an increased humoral reactivity. Such an increased humoral reactivity is indicative of subjects at higher risk of graft rejection or dysfunction, an increased risk of transfusion reaction, an increased risk of allogenic fetus rejection, or to an increased risk of auto-immune disease activity because of autoantibodies.

As used herein, a decrease in CX3CR1 and/or in CD16 cell's expression corresponds to a decrease of at least 10 % compared to control, preferably to a decrease of at least 20 %, and more preferably of at least 50 %.

The method of the invention can be used in the following applications : Transplantation (including cross match incompatibility, recipient monitoring, graft function, acute and chronic rejection, vascular disorders), transfusion (including Cross match incompatibility Platelet transfusion inefficiency, refractory response to platelet transfusion therapy, Transfusion Related Acute Lung Injury (TRALI) , LBP (Labile blood product) selection in transfusion settings, Post transfusional shock incident Feto-maternal incompatibility Newborn low platelets), diagnostic and prevention in reproductive medicine (including implantation failure, Infertility issues with etiology of auto antibodies, vascular disorders, foetal growth retardation, preeclampsia), autoimmune disease: diagnostic prognostic and follow-up, in particular as example test of autoantibodies driven vasculopathy, PTT purpura, chronic and acute immune thrombocytopenic purpura, Anti-phospholipid syndrom, lupus, vascular inflammatory diseases, Neurological diseases, monitoring of HIV infection and treatment, host related therapeutic efficiency, for example in auto and alloimmune processes, cancer and viral diseases, therapeutic monotoring, design of novel therapeutic protocols aiming at limiting humoral deleterious effects of allo or auto antibodies, evaluation of novel anti cancer or anti viral therapies using antibody or Fc based compounds agonist or antagonist to specifically eliminate tumor cells expressing specific antigens, Vaccination protocoles, Indication of vaccination efficacy, design of vaccination protocol's evaluation, monitoring of Arthrosclerosis, HIV or viral infection susceptibility or evolution, Viral serology evaluation, and Cellular and tissular regenerative medicine.

Preferably, the method of the invention can be used with a transplanted subject or a subject that has to be transplanted, with a transfused subject or with a subject that has to be transfused, with an infertile or pregnant female or with a subject suffering from an auto-immune disease.

As used herein, cells expressing target antigen of the present invention can be simply identified by the skilled person according to the pathological context. In particular these cells expressing target antigen can be Lymphocytes, Donor Spleen cells, Endothelial cells, cell therapy product, progenitor cells, Platelets, Primary and immortalized cell lines, Transfected cells, Transfected cell lines transfected with glycoprotein platelets antigen shRNAi silencing elements, Neutrophilic polynuclear cells, NPC panel, Transfected cell lines transfected with HNA antigens shRNAi silencing elements, Red blood cells, Any autoantigen expressing cell, Cell expressing vaccination target antigenic determinant, Fractalkine expressing or secreting cell, and virally Infected cells.

In particular, the method according to the present invention is as follows:

| Cells expressing target antigen | Serum or medium containing therapeutic Fc compound or compounds interfering with the fractalkine CX3CR1 pathways | NK cells | Applications |
|---|---|---|---|
| Lymphocytes Donor Spleen cells Endothelial cells Platelets Primary and immortalized cell lines Transfected cells | Anti HLA or anti MIC Alloantibodies | NK cells PBMC NK cell lines and NK cells transfected with CD16 and CX3CR1 variants | Transplantation Cross match incompatibility Platelet transfusion inefficiency Transfusion Related Acute Lung Injury (TRALI) : diagnostic and prevention in primary pregnancy LBP (Labile blood product) selection Post transfusional shock incident Infertilty Pregnancy loss |
| Donor's platelet Platelet panel Transfected cell lines transfected or not with glycoprotein platelets antigen shRNAi silencing elements | Alloantibodies anti HPA | NK cells PBMC NK cell lines and NK cells transfected with CD16 and CX3CR1 variants | Newborn low platelets Foetomaternal incompatibility diagnostic Platelet transfusion inefficiency PTT purpura |
| Neutrophilic polynuclear cells NPC panel Transfected cell lines transfected with HNA antigens shRNAi silencing elements | Anti HNA | NK cells PBMC NK cell lines and NK cells transfected with CD16 and CX3CR1 variants | TRALI diagnostic and selection of multiparous Foetomaternal incompatibility (IFM) |
| Red blood cells Red blood cells transfected with antigens or shRNAi silencing elements | Alloantibodies against red blood cells | NK cells PBMC NK cell lines and NK cells transfected with CD16 and CX3CR1 variants | IBP selection in transfusion settings |
| Lymphocytes Donor Spleen cells Endothelial cells Platelets Primary and immortalized cell lines Transfected cells | Non HLA, non HNA non HPA Alloantibodies | NK cells PBMC NK cell lines and NK cells transfected with CD16 and CX3CR1 variants | Transplantation Pregnancy disorders diagnostic and treatment |
| Any autoantigen expressing cell Endothelial cells Neutrophils Polynuclear Lymphocytes Donor Spleen cells Platelets Primary and immortalized cell lines Transfected cells | Systemic and organ Auto antibodies directed against constitutive or pathogen induced cell antigens | NK cells PBMC NK cell lines and NK cells transfected with CD16 and CX3CR1 variants | Any Autoimmune disease: diagnostic prognostic and follow-up In particular as example test of autoantibodies driving vasculopathy, SAPL, lupus, vascular inflammatory diseases Pregnancy loss Infertility with etiology of auto antibodies Transplantation (stress induced antigens such as MIC fractalkine) Inflammatory diseases |

(continued)

| Cells expressing target antigen | Serum or medium containing therapeutic Fc compound or compounds interfering with the fractalkine CX3CR1 pathways | NK cells | Applications |
|---|---|---|---|
| cell expressing antigen | Therapeutic antibodies Fc<br><br>receptor bearing compound: humanized antibodies, IvIg, Antithymoglobulin , chimeric recombinant proteins | NK cells PBMC<br><br>NK cell lines and NK cells transfected with CD16 and CX3CR1 variants | Host related Therapeutic efficiency<br>Therapeutic follow-up Indication and efficiency of treatment (IvIG and infertility) Design of new therapeutic protocols aiming at limiting humoral deleterious effects of allo or auto antibodies Evaluation of novel anti cancer therapy using antibody or Fc bases compounds agonist or antagonist to specifically eliminate tumor cells expressing specific antigens |
| Cell expressing vaccination target antigenic determinant | Serum during the follow up of vaccination strategies | NK cells PBMC NK cell lines and NK cells transfected with CD16 and CX3CR1 variants | Vaccination Indication of vaccination efficacy Design of vaccination protocol's evaluation |
| Fractalkine expressing or secreting cell | Serum or Therapeutic agents potential to interfere with the CX3CR1 fractalkine pathway | NK cells PBMC NK cell lines and NK cells transfected with CD16 and CX3CR1 variants | Monitoring of Arthrosclerosis Cancer Neurological diseases Vasculopathy Preeclampsia HIV or viral infection susceptibility or evolution |
| Infected cell presenting membrane bound viral antigen | Host serum natural anti viral antibodies | NK cells PBMC NK cell lines and NK cells transfected with CD16 and CX3CR1 variants | Viral serology evaluation |
| Progenitor cells | Host serum antibodies or therapeutic compound aiming to boost cellular therapeutic effect | Host immune cells | Monitoring and design of cellular therapy protocols notably in regenerative medecine |

According to a fifth preferred embodiment, said subject is a subject that has to be transplanted and the method of the invention is for determining the risk of graft dysfunction, to anticipate endothelial lesions and alloantibodies mediated graft rejection of said subject.

Advantageously, the cells expressing target antigen are cells of the donor or graft cells expressing alloantigens of said donor such as HLA antigens, preferably said cells expressing target antigen are cells of the donor.

According to a sixth preferred embodiment, said subject is a transplanted subject and the method of the invention is for determining the risk of chronic or acute graft rejection, graft dysfunction or cardiovascular complications resulting from the immunization status of said subject.

Advantageously, the cells expressing target antigen are cells expressing stress or pathogen induced auto antigens of said donor such as MIC A or inflammation antigens, preferably said cells expressing target antigen are cells of the donor.

Said subject may be treated or not with immunosuppressive agents.

The steps (i) and (ii), and eventually (iii), can be repeated once or several times so as to follow the risk of acute or chronic graft rejection in said subject because of antibodies directing humoral responses toward the graft .

According to a seventh preferred embodiment, said subject is a transfused subject and the method of the invention is for determining the risk of transfusion reaction in said subject because of antibodies such as auto or alloantibodies.

Said subject may be treated or not with immunosuppressive agents.

Advantageously, the cells expressing target antigen are cells of the donor, such as donor's platelet, or cells expressing alloantigens of said donor such as HPA antigens, preferably said cells expressing target antigen are cells of the donor.

The steps (i) and (ii), and eventually (iii), can be repeated once or several times so as to follow the risk of transfusion reaction in said subject because of alloantibodies.

The obtained prognosis can be thus used so as to adapt the immunosuppressive agents' regimen.

According to an eighth preferred embodiment, said subject has to be transfused and the method of the invention is for determining the risk of transfusion reaction in said subject because of antibodies such as auto or alloantibodies.

Advantageously, the cells expressing target antigen are cells of the donor, such as donor's platelet, or cells expressing alloantigens of said donor such as HPA antigens, preferably said cells expressing target antigen are cells of the donor.

According to a ninth preferred embodiment, said subject is a suffering from an auto-immune disease and the method of the invention is for determining the risk of evolution of said subject auto-immune disease because of autoantibodies.

As an example of theses diseases, one can cite vasculopathy such as Wegener and Kawasaki disease, SAPL, lupus, vascular inflammatory diseases, Neurological diseases, and arthrosclerosis.

Said subject may be treated or not with immunosuppressive agents, or therapeutic antibodies.

Advantageously, the cells expressing target antigen are any cells expressing any autoantigen.

The steps (i) and (ii), and eventually (iii), can be repeated once or several times so as to follow the risk of evolution of said subject auto-immune disease because of autoantibodies or to monitor treatment of disease.

The obtained prognosis can be thus used so as to adapt the immunosuppressive agents' regimen or treatment indicated to lessen humoral reactivity of the host (anti CD20 RITUXIMAB® therapy, plasma exchange, Intravenous immunoglobulins).

According to an eighth embodiment, said subject is to be transfused and the method of the invention is for determining the infused blood product in said subject.

According to a ninth embodiment, said subject is a female at risk for implantation failure and the method of the invention is for determining the risk of fetus rejection for said pregnant female because of auto or alloantibodies

Said subject may be treated or not with immunosuppressive agents or treatment indicated to lessen humoral reactivity.

The steps (i) and (ii), and eventually (iii), can be repeated once or several times so as to follow the risk of implantation failure or allogenic fetus rejection.

The obtained prognosis can be thus used so as to adapt the immunosuppressive agents' regimen or treatment indicated to lessen humoral auto or allo reactivity reactivity of the female toward fetus or partner and favor pregnancy success.

Advantageously, the cells expressing target antigen are cells of the fetus or cells expressing alloantigens of said fetus such as HLA antigens.

According to a tenth embodiment, said subject is a women experiencing immune related disorders in the reproductive field (infertility, implantation failure, vascular disorders, fetal growth retardation, preclampsia, miscarriage or foetal loss) the method of the invention is for determining the risk of reproductive failure for said pregnant female because of auto or alloantibodies.

Said subject may be treated or not with immunosuppressive agents or treatment indicated to lessen humoral reactivity.

The steps (i) and (ii), and eventually (iii), can be repeated once or several times so as to follow the risk of immune related disorders.

The obtained prognosis can be thus used so as to adapt the immunosuppressive agents' regimen or treatment indicated to lessen humoral auto or allo reactivity reactivity of the female toward fetus or partner.

Advantageously, the cells expressing target antigen are cells of the fetus or cells expressing alloantigens of said fetus such as HLA antigens.

According to a eleventh embodiment, said subject is submitted to treatment aiming to reduce humoral activity (Intravenous immunoglobulins, plasma exchange, anti B lymphocyte therapy, immunosuppressive regimen, therapeutic antibodies, agonist or antagonist therapy targeting FcR or CX3CR1 pathways) and the method of invention is to determine the potential efficiency before treatment and/or to monitor the efficiency of the treatment in said subject.

According to a twelfth embodiment, said subject is submitted to a treatment based on agonist or antagonist therapy targeting FcR, IgG or CX3CR1 pathways to limit any disease including cardiovascular, autoimmune diseases, neurologic diseases, cancer, viral infection and said method is to refine indication and efficiency of a treatment in said subject.

According to a thirteenth embodiment, said subject is submitted to vaccination therapy or therapy using exosome or microparticles expressing target antigens as vehicles of functional activities that modulate fractalkine-CX3CR1 and Fc-FcR pathways.

In a second aspect, the present invention refers to a kit for determining a subject cellular humoral activity, preferably a subject cellular humoral activity directed against an antigen, comprising (i) at least one mean for determining the expression profile of CX3CR1 gene in effector cells expressing FCR (preferably FCRG) and CX3CR1, preferably in lymphocytes, $CD3^-CD56^+$ NK cells and monocytes, most preferably in $CD3^-CD56^+$ NK cells.

Such a mean is well known from the skilled person and has been disclosed previously.

Advantageously, such a mean is an antibody directed against the CX3CR1 protein. Preferably, such a mean is an antibody directed against the CX3CR1 protein enabling the detection by flow cytometry of the CX3CR1 protein expressed at NK cells' surface, more specifically at $CD3^-CD56^+$ NK cells' surface.

In a preferred embodiment, the kit of the invention further comprises (ii) at least one mean for determining the expression profile of FCR, preferably FCRG gene, most preferably of CD16 gene, in effector cells, preferably $CD3^-CD56^+$ NK cells.

Such a mean is also well known from the skilled person and has also been disclosed previously.

Advantageously, such a mean is an antibody directed against the CD16 protein. Preferably, such a mean is an antibody directed against the CD16 protein enabling the detection by flow cytometry of the CD16 protein expressed at NK cells' surface, more specifically at $CD3^-CD56^+$ NK cells' surface.

In still a preferred embodiment, the kit of the invention further comprise at least one cell line derived from NK cells.

In another embodiment, the kit is made up of instructions for carrying out any of the methods described herein. The instructions can be provided in any intelligible form through a tangible medium, such as printed on paper, computer readable media, or the like.

As used herein, the term "kit" refers to any delivery system for delivering materials. In the context of reaction assays, such delivery systems include systems that allow for the storage, transport, or delivery of reaction reagents (e.g., antibodies, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kits can also include one or more reagents, buffers, hybridization media, antibodies, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

Still a further aspect of the present invention refers to the use, for determining a subject cellular humoral activity directed against an antigen, of the abovementioned kit.

In the following, the invention is described in more detail with reference to methods, antibodies, amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

## EXAMPLES

*Example 1: NK activation by Fc humanized therapeutic compound such as Rituximab is correlated with CX3CR1 and CD16 expression decrease at NK cell's surface*

Effector cells: Human peripheral blood mononuclear cells (PBMC) were isolated from blood of healthy donors using density gradient centrifugation on Ficoll-Hypaque.

Fc Compound is the therapeutic preparation of CD20 humanized antibodies rituximab used at a concentration of 10μg/ml

Introduced target cells titrating anti CD20 cellular humoral activity are immortalized B-EBV transformed cell lines. CD20 positive B lymphocytes found in PBMC effector cells preparation can also evaluate efficiency of rituximab in mediating activation host NK cytokine production and cytotoxic activity against them, as evaluated here by rituximab induced intracytoplasmic INF-γ production and granule exocytosis (CD107a/lamp-1) in phenotypically defined CD3-CD56+ NK cell subsets analyzed by flow cytometry within PBMC.

As an example NK activation induced toward B lymphocyte targets are in the presence of rituximab and of target cells expressing CD20, PBMC were plated at 0.5 $10^6$ cells/well simultaneously with immortalized B-EBV transformed cell lines at 0.5 $10^6$ cells/well and with rituximab at 10μg/ml final concentration.

CD16, CX3CR1, and NK cell activation evaluated by CD107a/Lamp-1 and IFN-γ NK cells' expression were simultaneously tested

As controls, PBMC were plated at 0.5 $10^6$ cells/well simultaneously or not with immortalized B-EBV transformed cell lines at 0.5 $10^6$ cells/well or incubated alone with Rituximab at 10μg/ml final concentration.

Table 4 shows the ability of rituximab to induce variations in mean fluorescence of CD16 staining in CD16 positive CD3-CD56+ NK cells, % CX3CR1 in CD3-CD56+ NK cells, % of CD3-CD56+ expressing CD107a/lamp1 and intracytoplasmic IFN-γ after incubation of effector PBMC in the presence or in the absence of Rituximab and/or of immortalized B-EBV transformed cell lines.

Table 4

| | CD16 (MFI) in CD3-CD56+ | % CX3CR1 in CD3-CD56+ | % CD107a/lamp Expression in CD3-CD56+ | % of intracytoplasmic IFN-γ in CD3-CD56+ |
|---|---|---|---|---|
| TEST against B-EBV transformed cell lines PBMC + B immortalized B-EBV transformed cell lines + RITUXIMAB® | 3 | 59 | 41 | 17 |
| Control 1: PBMC alone | 40 | 88 | 4 | 1 |
| Control 2 PBMC + immortalized B-EBV transformed cell lines | 35 | 89 | 5 | 1 |
| TEST Autologous PBMC + RITUXIMAB® | 20 | 77 | 24 | 8 |

The results show that, as observed by analyzing CD107a/lamp-1 expression and IFN-γ production, whereas immortalized B-EBV transformed cell lines do not activate NK cells in PBMC, a strong activation of these NK cells in PBMC is observed in the simultaneous presence of rituximab and immortalized B-EBV transformed cell lines.

This NK cells activation is correlated with a decrease of CD16 and CX3CR1 expression. In the presence alone of rituximab, the humoral activity of B lymphocytes found in PBMC also induces decrease of CD16 and CX3CR1 expression associated with NK activation.

Scoring of rituximab induced humoral cellular activity against B-EBV transformed B cells contained in the preparation can be expressed as rituximab induced variation in expression CD16 and CX3CR1 markers in reference to that evaluating rituximab induced activation of NK cell function.

A first readout of the CHAT test is the quantification of the subject's potential to generate a NK mediated cellular humoral response towards autologous B lymphocytes after treatment by the therapeutic Fc coumpound rituximab directed against CD20 antigens.

A second level of the chat test readout is the introduction in the test of a fixed number of exogeneous B cell line targets expressing CD20 to further enhance specificity and sensitivity of the test by providing higher amounts of CD20 target antigens.

Score 1: CHAT scoring of NK mediated Rituximab response against autologous CD20 expressing B lymphocyte cells found within the PBMC preparation % CD107 (Test auto -CR1)+ %IFN(Test auto-CR1)= (40-20)+(8-1)= 27

**CHAT test readout scores**: % CX3CR1 (CR1-test auto) and Mfi CD16 (CR1-test auto) these indexes can be used separately or combined by summing.

Score 2 : CHAT Fonctional scoring of rituximab induced NK activation versus exogeneous B cell lines

% CD107 (Test -CR2)+ %IFN (test-CR2) = (41-5)+(17-1)= 52

**CHAT test readout scores**: % CX3CR1 (CR2-test) and Mfi CD16 (CR2-test) = ΔCX3CR1 and Δ mfi CD16, these indexes can be used separately or combined by summing.

In a preferred indexing of CHAT, control values are set as 100% and rituximab induced variations (decrease in %CX3CR1 and MfiCD16 and fold increase of NK cell degranulation and IFN production) are expressed in % of variation relative to controls. To evaluate rituximab induced activity against autologous B lymphocytes found within PBMC of the host, values of the test are obtained when PBMC are incubated with Rituximab in reference to those evaluated in PBMC controls without rituximab. To evaluate rituximab induced activity against exogeneous B-EBV transformed cell lines, values of the test are obtained when PBMC are incubated with Rituximab and B-EBV cell lines and scored in reference to those evaluated in PBMC controls incubated with B-EBV targets but without rituximab.

| | | |
|---|---|---|
| **CD16 Mfi** | 1 PBMC | 40 |
| **against auto B Ly** | T B Ly PBMC+ RIT | 20 |
| | DELTA Mfi CD16 | 20 |
| | **% Ritux induced decrease** | **50** |
| **against BEBV cell target** | PBMC+ B-EBV | 35 |
| | PBMC+BEBV+RIT | 3 |
| | DELTA Mfi CD16 | 32 |
| | **% Ritux induced decrease** | **91** |
| | | |
| **%CX3CR1** | 1 PBMC | 88 |
| **against auto B Ly** | T B Ly PBMC+ RIT | 77 |
| | DELTA %CX3CR1 | 11 |
| | **% Ritux induced decrease** | **12,5** |
| **against BEBV cell target** | PBMC+ B-EBV | 89 |
| | PBMC+BEBV+RIT | 59 |
| | DELTA %CX3CR1 | 30 |
| | **% Ritux induced decrease** | **34** |
| | | |
| **CD107** | 1 PBMC | 4 |
| **against auto B Ly** | T B Ly PBMC+ RIT | 24 |
| | DELTA CD107 | 20 |
| | **fold increase after Rituximab** | **6** |
| **against BEBV cell target** | PBMC+ B-EBV | 5 |
| | PBMC+BEBV+RIT | 41 |
| | DELTA CD107 | 36 |
| | **fold increase after Rituximab** | **8,2** |
| | | |
| **IFN-g** | 1 PBMC | 1 |

(continued)

| | | |
|---|---|---|
| **against auto B Ly** | T B Ly PBMC+ RIT | 8 |
| | DELTA IFN-g | 7 |
| | **% Ritux induced decrease** | **8** |
| **against BEBV cell target** | PBMC+ B-EBV | 1 |
| | PBMC+BEBV+RIT | 17 |
| | DELTA IFN-g | 16 |
| | **fold increase after Rituximab** | **17** |

These scores can be combined or used independently to assay RITUXIMAB® or any IgFc mediated host cellular humoral activation directed against target antigen. Values obtained by CHAT evaluation of the Rituximab induced NK cell humoral activity of the host against its B cells is dependent of the number of blood B cells found in the host PBMC preparation and of host CD16 level of expression within NK cells. It can predict a subject capacity to respond to a treatment that consist of a humanized antibody or Fc fragment.

CHAT scoring of rituximab induced NK cell activity against B-EBV transformed cell lines analyzed in reference to PBMC + Target in 58 healthy individuals resulted in a median decrease of CD16mfi of 90% and a 63% decrease in %CX3CR1.

*Example 2: Value of the CHAT test for comparative analysis of cellular humoral activation induced by anti CD20/ rituximab and serum of a transplant recipient containing HLA-A2 alloreactive antibody toward B-EBV transformed cell lines expressing CD20 and HLA-A2 target antigens: correlation of NK cell activation markers and antibody induced decrease CX3CR1 and CD16 expression in NK cell surface*

Binding of antibodies found in serum to their target can be assessed by flow cytometry. Briefly, B-EBV transformed cell lines expressing HLA-A2 are used as target cells. Antibody binding to target is analyzed after incubation with different anti-HLA-A2 or not sera used at 20% for 20 minutes then washed in PBS. Detection is made using a Fab'2 anti-human IgG (Fc) antibody fragment conjugated to PE (Beckman Coulter IM0550). Labeling is obtained after incubation for 15 minutes at room temperature protected from light and after 1 wash step cells are resuspended in 500μl PBS 2%SVF. Data acquisition and analysis is performed on Beckman coulter FC500 cytometer with CXP software. Analysis is done through MFI analysis gating of stained target cells.

To perform the CHAT test, CD107n/lamp-1, CM16 and CX3CR1 NK phenotype is assessed within host PBMC by flow cytometry as previously described to constitute the signature of NK response to coculture with cells expressing target antigen and antibodies. Control reference conditions check target cell or control serum induced modulation of NK cell activation markers. Negative Control serum consists of autologous host serum or serum without detectable anti class I and class II HLA antibodies by luminex assay. Other control conditions evaluate the capacity of antibodies or sera to activate effector NK cells against PBMC cells in absence of exogenous introduced target cells.

Briefly, $110^6$ PBMC are incubated for 4 hours at 37°C with or without $110^6$ EBV transformed B lymphocyte cell lines and with or without test serum or autologous serum or rituximab at 10μg/ml final concentration). This incubation is done is presence of Golgi Stop (Becton Dickinson 554724), and CD107-PC5 (Becton Dickinson 555802). Cells are then washed and labeled with CD3-ECD (Beckman Coulter A07748), CD16-FITC (Beckman Coulter IM0814), CD56-PC7 (Beckman Coulter A21692) and CX3CR1 (MBL D070-3) for 15 minutes at room temperature protected from light. After 1 wash step, cells are resuspended in 500μl PBS 2%SVF.

Data acquisition and analysis is performed on Beckman coulter FC500 cytometer. NK population is gated using CD3/CD56 labeling (CD3-CD56+ population). CD16, CX3CR1 and CD107a/LAMP-1 expression is analyzed on host NK effector cells within PBMC.

Deregulation activity of NK cells resulting from anti CD20 or anti HLA-A2 serum was indexed by measure of CD107a/LAMP-1 induction observed in NK cells found in PBMC after stimulation of HLA-A2 and CD20 expressing target cells in presence of autologous serum, autologous serum and rituximab or 20% of serum of a transplanted patient with humoral rejection in which anti HLA-A2 activity was characterized by Luminex assay.

Scoring of Rituximab induced NK cell humoral activation against target CD20 antigen was achieved by comparing values obtained in test conditions to values obtained in effectors cells incubated in presence of HLA-A2 and CD20 expressing B lymphocyte target cells and autologous serum without adding rituximab and was resumed as shown in Table 6.

A negative human serum (Serum GAU) in which no humoral activity was expected considering absence of HLA antibodies, does not increase NK cell activation by the CHAT test. In contrast, CHAT assay shows that Rituximab or anti HLA-A2 have similar potential in activation of NK cells towards EBV-transformed cell lines expressing Target antigens and result in respectively a 18 and 17 scoring of CD107a/LAMP-1 expression , a variation of %CX3CR1 score of 30 and of 43,8 and a variation of CD16 mfi score of 46,6 and of 35,5

CHAT scoring of % variation was obtained comparing values in test (including , targets +PBMC effector cells + HLA-A2 B_EBV targets + Serum or Rituximab) and expressed as relative decrease in reference to values observed (considered as 100%) when control PBMC and B-EBV target cells were incubated without serum

| | **Variation TEST Ritux vs Control B-EBV+ PBMC** | **variation TEST anti A2 serum vs Control B-EBV A2 + PBMC** |
|---|---|---|
| **decrease in CX3CR1 Score** | **42** | **50** |
| **decrease in Mfi CD16 Score** | **90** | **84** |
| CD107-Lamp Fold induction | 3 | 2 |

These results show that CHAT scoring of serum induced phenotypic variations of CX3CR1 and CD16 within the CD3-CD56+ NK cell effector subset of host PBMC can evaluate the capacity of a subject's serum to mediate host cellular humoral activation.

<u>*Example* 3: *Cellular humoral NK activation (CD107a/LAMP-1 and IFN-γ production) induced by serum of transplant recipient toward spleen donor cells (Cross match) is correlated with a CX3CR1 expression decrease at NK cell's surface*</u>

As described previously, NK cell phenotype assessed within PBMC by flow cytometry constitute the signature of cellular humoral response to coculture with target cells and sera. Here target cells are chosen to reflect cells of a donor organ to be transplanted (spleen donor cells) while serum and effector cells are chosen to reflect the host response of the organ recipient to be transplanted. Aim of the test is to perform a cross match test evaluating the cellular humoral response of a transplant recipient to donor cell target antigens that may be responsible of organ rejection. Control reference conditions include test of effector serum in presence or in absence of target donor human spleen cells depleted in NK cells (Depletion of spleen NK cells that may interfere with testing of recipient CHAT scoring is obtained after CD16 positive magnetic selection, in this case using Miltenyi Biotec MACS CD16 microbeads Ref 130-045-701). Luminex assay of serum specific anti HLA-A antibodies and LCT activity of tested sera were obtained in independent evaluations and sera were analyzed in a blind test to assay cross match reactivity of serum induced humoral activity in PBMC cells of a healthy donor representative of the host recipient cells. Control sera in which no reactivity for HLA specificities were detectable by luminex antibodies were used to score negative threshold of HLA negative serum and rituximab activity against B lymphocytes present in spleen donor cell preparation was used to score a positive cellular humoral activity. Briefly, $110^6$ effector cells (recipient PBMC) were incubated for 4 hours at 37°C with or without $110^6$ target (human spleen cells), in presence or in absence of serum to be evaluated. This incubation was performed in presence of Golgi Stop (Becton Dickinson 554724) allowing evaluation of granule exocytosis, and CD107-PC5 (Becton Dickinson 555802) and Intracellular IFN-g staining of CD3-CD56= NK cells (Becton Dickinson). Cells were washed and labeled with CD3-ECD (Beckman Coulter A07748), CD16-PE (Beckman Coulter), CD56-PC7 (Beckman Coulter A21692) and CX3CR1-FITC (MBL) for 15 minutes at room temperature protected from light. After 1 wash step, cells were resuspended in 500μl PBS 2%SVF.

Data acquisition and analysis was performed on Beckman coulter FC500 cytometer after gating of the NK effector cell subset within PBMC using CD3/CD56 labeling (NK cells refer to the CD3-CD56+ population). Serum induced variations in IFN-g intracellular staining and CX3CR1 and Lamp/CD107a/LAMP-1 cell surface expression were further analyzed on NK host effector subsets tested in presence or in absence of spleen target cells.

Assay of HLA-A2 serum capacity to modulate CD16 Mfi, %CX3CR1 expression and NK cell activation (CD107A/LAMP-1 and IFN-γ expression) towards HLA-A2 donor spleen cells is depicted below:

The results are shown in Figure 1.

Various serum were then evaluated for their capacity to induce humoral cell activation of the recipient toward HLA-A2 and HLA-A3 spleen donor cells

| | Anti-HLA characteristics of serum | | DELTA CX3CR1 score | DELTA IFN-γscore | DELTA CD107a/ LAMP-1 score |
|---|---|---|---|---|---|
| | luminex ACAL detection | CDC | | | |
| RITUXIMAB 10 μg ml-1 | na | na | 5,1 | 7,77 | 5,09 |
| IgG purified from SERUM Del 10 μg.ml-1 | HLA-A2 | positive | 11,8 | 3,26 | 17,4 |
| 10% SERUM Boo | anti HLA-A2 | positive | 10,6 | 18,16 | 13,1 |
| 10% SERUM Tol | anti HLA-A2 | positive | 7,8 | 9,88 | 2,2 |
| 10% SERUM Bse | anti HLA-A2, A28, A9 | positive | 8,47 | 9,14 | 11,3 |
| SERUM 1 Bar | anti HLA-A2, A28 | negative | 0,9 | 2,32 | 5,5 |
| SERUM 2 Bar | anti HLA-A2, A29 | negative | 2,89 | 0,7 | 0,9 |
| SERUM Car | anti HLA-A2, A68 | negative | 20,55 | 6,69 | 10,2 |
| SERUM 1 Koz | neg | negative | 2,4 | 3,96 | 4,4 |
| SERUM 2Koz | pos HLA-A3 | negative | 2,6 | 3 | -4 |
| 10+ ACAL negative Human CTL serum | negative | negative | 1 | 6,58 | 4,8 |

Graphic representation testing of serum induced variations of CX3CR1, CD107a/LAMP-1 and IFN-γ expressions on effector cells evaluated in absence or in presence of donor spleen target cells.

All positive CDC sera and rituximab were evaluated positive with regards to CHAT assay of host cellular humoral activity towards spleen cells.

In contrast, most sera tested as negative by CDC despite anti HLA-A2 or HLA-A3 antibodies detected by luminex which should recognize spleen cells were scored as negative (Bar, Koz) in the CHAT test. This is probably the reflect of low level of anti-HLA-A2 or HLA-A3 allo-antibodies in sera that can be detected by the sensitivity of luminex assay while these antibodies appear to have no potential to exert functional deleterious damage on target cells, nor by CDC or ADCC. Interestingly, one of the sera (Car) scored as HLA-A2 positive in luminex assay but negative in LCT was shown to exert CHAT activity against spleen antigens. This suggest that CHAT can reveal functional pathogenic effects of antibodies in mediating target injury that would refine the sole description of HLA specificities detected by luminex and would have been missed by the CDC assay. In addition, luminex only addresses detection the presence of anti HLA and anti -MICA antibodies while CHAT test gives information both on seric humoral activity directed against any target antigen but also accounts for the recipient's efficiency to elicit an antibody dependent immune response towards donor targets. We thus propose that CHAT testing be associated to luminex assay of recipient-donor cross match in organ transplantation. The importance of the test may also prove determinant to detect non HLA deleterious cellular humoral activity of a host, which is thought to determine organ dysfunction and rejection and are currently missed by conventional assays.

*Example 4: Cellular humoral NK activation induced by serum of an infertile women toward endothelial allogeneic targets is correlated with CX3CR1 and CD16 expression decrease at NK cell's surface and target endothelial cell MP release*

Effector host PBMC are isolated from whole blood after ficoll gradient.

Human endothelial microvascular HLA-A2 cell lines are used as targets.

Serum is that of an infertile woman tested before initiation of in vitro fertilization procedure, the issue of IVF resulting in implantation failure. Detection of an anti endothelial binding activity in the serum of infertile women host is revealed by flow cytometry using human endothelial cells incubated in presence of 10% serum and staining of antibody binding using a Fab'2 anti human Fc PE antibody in reference to background binding of a control male serum, with no humoral component. Sera are used after decomplementation for 30min at 58°C and centrifuged before use in the test

Assay of the NK mediated humoral lytic activity of the serum towards endothelial cells expressing target antigen

Lysis of target cells was evaluated both as a measure of survival of CMFDA labeled endothelial cell targets (ECT) and release of labeled endothelial cell derived microparticles (EMP).

**CMFDA Cytotoxicity assay**

24h prior to cytotoxicity assay, HLA-A2 positive HRGEC (Human Renal Glomerular Endothelial Cells) were seeded onto 96-well plate at $10\times10^3$cells/well in EGM-2 medium and allowed to attach for 6h at 37°C in 5% CO2.

The cells were then stimulated with IFN-$\gamma$ (50ng/ml) and TNF-$\alpha$ (20ng/ml) for 20h.

For cytotoxicity assay, target cells (HLA-A2 HRGEC and late EPC)
were labelled with 5$\mu$M Cell Tracker™ Green CMFDA (Invitrogen) for 15 min at 37°C in 5% CO2 in the dark. The CMFDA was removed and the cells were further incubated with RPMI medium containing 5% FCS for 30 min to stop the CMFDA staining.

Then HLA-A2 negative PBMC effector cells were added at effector: target (E: T) ratios of 100:1 and incubated with target cells overnight in the presence of 20% of anti-HLA-A2 positive serum or 20% of anti-HLA-A2 negative control serum.

After incubation, medium containing effector cells was removed, target cells were washed with PBS with Ca2* and Mg2* and the fluorescence of resting non-damaged targets was measured using multi-plate cell reader Cytofluor® (PerSeptive Biosystems). Assays were performed in triplicate, and data were expressed as percent of cytotoxicity:

$$\%\ \text{Cytotoxicity} = \left(\frac{\text{IF targets} - \text{IF (targets+effectors)}}{\text{IF targets}}\right) \times 100$$

Each well was also analysed by epi-f luorescence microscopy on a inverted microscope Nikon EclipseTM TE2000-U with a Plan Fluor 4x /0,13 objective, images were acquired using NIS elements AR software.

**Assay of serum induced endothelial cell Microparticle release**

24h prior to test, HLA-A2 positive HRGEC (Human Renal Glomerular Endothelial Cells) were labelled with Lipophilic Tracer DiD (Molecular Probes, Invitrogen). Briefly, $1\times10^6$ cells were incubated with 8$\mu$g/ml of DiD lipophilic tracer in 1ml of RPMI medium without serum for 1H at 37°C in 5% CO2 in the dark. After incubation cells were washed 4 times with RPMI medium without serum,seeded onto 96-well plate at $10\times10^3$cells/well in EGM-2 medium (Lonza) and allowed to attach for 6h at 37°C in 5% CO2. The cells were then stimulated with IFN-$\gamma$ (50ng/ml) and TNF-$\alpha$ (20ng/ml) for 20h. HLA-A2 negative PBMC effector cells were added at effector: target (E: T) ratios of 0:1, 5:1, 50:1, 100:1 and incubated with target cells overnight in the presence of 20% of HLA-A2 positive serum or 20% of HLA-A2 negative control serum.

After an overnight incubation period, culture medium was harvested, centrifuged at 450g for 5 minutes to discard PBMC and cell debris and supernatants were collected for flow cytometry analysis of target cells-derived microparticles. For microparticles staining, 10$\mu$L of FITC-conjugated annexin V (Beckman Coulter) were added to 30 $\mu$L of supernatant. After 30 minutes of incubation at room temperature, samples were diluted in 500$\mu$L of annexin V binding buffer (Beckman Coulter), 30$\mu$L of counting beads with an established concentration of 960 beads/$\mu$L (Flowcount™ Fluorospheres, Beckman Coulter) were added to each sample in order to express microparticles counts as absolute numbers per microliter of supernatant.

The amounts of DiD/AnnexinV double positive microparticles with diameters between 0,5 and 1 $\mu$m in each supernatant were quantified on a Gallios cytometer (Beckman Coulter).

**Assay of IVF serum induced NK cell function (IFN production) in presence of endothelial cell targets**

24h prior to cytotoxicity assay, HLA-A2 positive HRGEC (Human Renal Glomerular Endothelial Cells) were seeded onto 96-well plate at 10×$10^3$cells/well in EGM-2 medium and allowed to attach for 6h at 37°C in 5% CO2.

The cells were then stimulated with IFN-γ (50ng/ml) and TNF-α (20ng/ml) for 20h.

HLA-A2 negative PBMC effector cells were added at effector: target (E: T) ratios of 5:1 and incubated with target cells overnight in the presence of 20% of HLA-A2 positive serum or 20% of HLA-A2 negative control serum. Protein transport inhibitor (Golgi stop, Becton Dickinson) was added in each well (final dilution 1/1500).

After an overnight incubation period, PBMC were harvested, washed with PBS 2% SVF and stained for 15 minutes at 4°C with 10μL of FITC-conjugated anti-human CX3CR1 (Cliniscience), 5μL of ECD-con jugated anti-human CD3 (Beckman Coulter), 5μL of PC5-conjugated anti-human CD16 (Beckman Coulter) and 5μL of PC7-conjugated anti-human CD56 (Beckman Coulter). Then cells were fixed and permeabilized using IntraPrep kit (Beckman Coulter) according to manufacturer's instructions. After permebilization; cells were incubated with 1,5μL of PE-conjugated anti-human IFN-© (Becton Dickinson) for 15 minutes at 4°C, washed with PBS 2% SVF and analysed by flow cytometry on a FC500 Cytometer (Beckman Coulter).

The results are shown in Figure 2.

*Example* 5: *antiendothelial antibodies can be found in intravenous IvIg therapeutic preparation and in sera of transplanted or infertile patients : rational to use the CHAT evaluation of the efficiency of IvIg therapeutic use*

The results are shown in Figure 3.

The therapeutic IvIg preparation (endobulin) or the anti endothelial seric IgG antibody binding of seras from kidney or heart transplant recipients transplant were evaluated by flow cytometry using unstimulated or stimulated microvascular glomerular cell targets (GMEC) or HUVEC endothelial cells.

Antibody binding was revealed by flowcytometry analysis using proteinA alexa fluor secondary reagent binding to the Fc fragment of human IgG present in the serum.

*Example* 6: *implication in regenerative medecine: Cellular humoral NK activation toward progenitor cell targets induced by antibodies present in the serum of a transplanted patient experimenting vascular humoral rejection is correlated with CX3CR1 and CD16 expression decrease at NK cells surface and target cell deri ved MP release and cell lysis*

Partners of the Test

Effector cells consist of PBMC isolated from a healthy blood donor after Ficoll gradient, and characterized as non HLA-A2.

Target cells consist of late endothelial progenitor cells (EPC) prepared from cord blood of an HLA-A2 positive donor. Late EPC were prepared using the following protocole: Mononuclear cells were isolated from human cord blood by centrifugation using lymphocyte separation medium (Eurobio). Monocytes were eliminated by overnight adhesion phase on plastic culture flask in RPMI 10% fetal calf serum at 37°C in 5% $CO_2$. Non adherent cells were plate on 24-well culture dishes coated with gelatin 0,2 % (Sigma- Aldrich) and cultured in endothelial basal medium EBM-2 (Lonza) supplemented with 2% fetal bovine serum and an endothelial growth supplement (EGM-2 SingleQuots, Lonza) containing human VEGF, epidermal growth factor, fibroblast growth factor-beta, insulin-like growth factor-1, hydrocortisone, ascorbic acid, and heparin. The medium was replaced every 3 days until the appearence of late-outgrowth EPCs colonies

Briefly, 2 × $10^5$ target cells are incubated with 20% serum for 20min at 4°C. After incubation , cells are washed with PBS 3% FCS and further incubated with protein A alexa Fluor (2.5mg/ml, binds Fc fragment) for 20 min at 4°C and flowcytometry assay (FC500) of labeled EPC cells that reveal specific binding of of antibodies present in the serum to targets. Control serum of a non immunized male healthy donor is used to assay specificity of serum binding to targets.

Serum used to evaluate cellular humoral activity is the serum of a transplanted patient tested to contain anti HLA-A2 humoral activity against graft after luminex assay. Binding of serum to the EPC -A2 target was controlled by indirect binding of the serum to EPC cell targets.

**Humoral cellular activity of a serum on EPC cell lysis**

Target cells (late EPC) were plated onto 96-well plate at 10000 cells/well in EGM-2 medium and were allowed to attach at 37°C, in 5% CO2 for 6h.

For cytotoxicity assay, target EPC cells were labelled with 5μM CellTracker™ Green CMFDA (Invitrogen) for 15 min at 37°C in 5% CO2 in the dark. After removal of CMFDA, the cells were further incubated with RPMI medium containing 5% FCS for 30 min to stop the CMFDA staining.

Then effector PBMC cells were added at effector: target (E: T) ratios of 100:1 PBMC and incubated ON at 37°C in 5% CO2 in the dark. 20% Patient's and control serum were added to the test containing either Effector and Targets or targets alone.

After incubation, medium containing effector cells was removed and after washing of target cells with washed with PBS with Ca2+ and Mg2+, target cell survival was measured by fluorescence assay of remaining target cells using multi-plate cell reader Cytofluor. Assays were performed in triplicate, and % of target cell lysis was estimated as follows [(IF of target cells without effectors - IF of target cells + effector cells) / (IF of target cells without effectors)] *100, thus evaluating percent of lysis/control sample (sample treated in the same condition without effector cells).

The results are shown in Figure 4.

Evaluation of humoral cellular activity of a serum by enumeration of EPC derived Microparticles (MP)

The test of MP is destinated to provide an alternative to the CMFDA assay of target cell lysis through the assay of MP release associated to target cell lysis.

To facilitate the read out of MP as deriving from target cells, targets cells were labeled by a cell tracker prior incubation with serum or effector cells. In a preferred assay, target cells were labelled 24h prior to test using the Lipophilic Tracer DiD (Molecular Probes, Invitrogen). For DiD labelling $1\times10^6$ cells were incubated with 8µg/ml of DiD lipophilic tracer in 1ml of RPMI serum free medium for 1H at 37°C in 5% CO2 in the dark. Then cells were washed 4 times with RPMI medium without serum and seeded in round bottom 96-well plate at 2x105/well and cultured for 12h in EGM-2 endothelial cell medium. In the test effector PBMC cells were added at effector: target (E: T) ratios of 100:1 PBMC and incubated ON at 37°C in 5% CO2 in the dark. 20% Patient's and control serum were added to the test containing either Effector and Targets or targets alone to evaluated minimal EPC MP release.

After incubation, cells were centrifuged at 300 g for 5min to discard cells, and 30 microliters of supernatants were further analyzed for quantification of DID+ annexin+ labelled target cell derived MP, gated on the basis of size identifying the 0.5-1 mM population using a Gallios flow cytometer (Beckman Coulter) after size standardization using 0.5, 0.9 and 3 µm diameters fluorescent beads (Megamix, BioCytex). To test the possibility to associate target cell DID labelling to a specific marker identifying origin of MP resulting from target cell lysis, we associated the CD31-PE labelled (PECAM) endothelial cell marker to that of DID identification of DID+CD31 double positive EPC derived MP.

FS and side scatter (SS) parameters were plotted after size standardization identification of MP using 0.5, 0.9 and 3 ⌠m diameters fluorescent beads (Megamix, BioCytex). Single staining controls were used to check fluorescence compensation settings and to set up positive regions. PMPs were here defined as dual-positive Phosphatidylserin (PS) +/CD31+ events, as seen in dual-color fluorescence plots after staining of DID positive MP with annexin V-FITC or CD31-PE antibodies. Thirty microliters of counting beads with an established concentration close to 1000 beads ⌠L□1 (Flow Count™ Fluorospheres; Beckman-Coulter) was added to each sample in order to express MP counts as absolute numbers per microliter of cell supernatant.

CX3CR1 and CD16 expression is down-regulated using serum containing humoral activity anti HLA-A2 activity against HLA-A2 EPC as compared to a control serum without anti HLA-A2.

The results are shown in Figure 5.

HLA-A2 EPC target cells were incubated ON with PBMC at a ratio of PBMC effector cells to target of 5:1 both in presence of a control serum or a serum from transplanted patient containing anti HLA-A2 reactivity evaluated by luminex (A2,A23,A24,A68,A69). We then analyzed the impact of control and Transplanted patient sera in with regard to engagement of CD16 and CX3CR1 when incubated with HLA-A2 EPC targets though phenotypic analysis of % CX3CR1 % CD16 and mfi CD16 CD3-CD56+ NK cells expression NK cells was performed.

| | Fold Reduction SERUM **Tranplant** / Serum control |
|---|---|
| %CX3CR1 | 3,4 |
| %CD16 | 10,4 |
| Mfi CD16 | 5,6 |

DELTA CX3CR1 score TEST SERUM-CTL serum= 61.5

The results are shown in Figure 6.

*Example 7: Pre stimulation of NK cells by anti CX3CR1 antibodies or fractalkine ligand can potentiate cellular humoral activity toward target endothelial porgenitor cells*

We tested the impact of a prestimulation of NK cells with CX3CR1 antibodies or soluble fractalkine to modulate NK cell activation towards endothelial cell progenitors (late EPCs) expressing fractalkine as a result of treatment by TNF-α and IFN-γ.

Generation and in vitro culture of Late outgrowth EPC

PBMC were isolated from human cord blood by centrifugation using lymphocyte separation medium (Eurobio).

Monocytes were eliminated by overnight adhesion phase on plastic culture flask in RPMI 10% fetal calf serum at 37°C in 5% $CO_2$.

Non adherent cells were plate on 24-well culture dishes coated with gelatin 0,2 % (Sigma- Aldrich) and cultured in endothelial basal medium EBM-2 (Lonza) supplemented with 2% fetal bovine serum and an endothelial growth supplement (EGM-2 SingleQuots, Lonza) containing human Vascular Endothelial Growth Factor, epidermal growth factor, fibroblast growth factor-beta, insulin-like growth factor-1, hydrocortisone, ascorbic acid, and heparin. The medium was replaced every 3 days until the appearance of late-outgrown EPCs colonies.

**Cytotoxicity assay**

Target cells (late EPC) were plated onto 96-well plate at 10000 cells/well in EGM-2 medium and were allowed to attach at 37°C, in 5% CO2 for 6h. Then cells were stimulated with TNF-$\alpha$ (20ng/ml) and IFN-y (50ng/ml) for 20h.

When transfectants were used as targets, 24h prior to cytotoxicity assay, late EPC were transfected with pCDNA 3.1(-) vector alone or with pCDNA 3.1(-) coding for membrane CX3CL1 and plated onto 96-well plate at 10000 cells/well.

For cytotoxicity assay, target cells (TNF-$\alpha$ IFN-$\gamma$ stimulated or transfected late EPC) were labelled with 5$\mu$M CellTracker™ Green CMFDA (Invitrogen) for 15 min at 37°C in 5% CO2 in the dark. The CMFDA was removed and the cells were further incubated with RPMI medium containing 5% FCS for 30 min to stop the CMFDA staining.

Then PBMC or purified NK cells were added at effector: target (E: T) ratios of 100:1 or 10:1 and 15:1. Effector and target cells were incubated for 4h at 37°C in 5% CO2 in the dark.

In some experiments, TNF-$\alpha$ IFN-$\gamma$ stimulated target cells were pretreated with 30$\mu$g/ml of goat anti-human CX3CL1 or with goat IgG control (R&D Systems) for 30 minutes at 37°C in 5% CO2. Effector cells were pretreated with recombinant human CX3CL1 (R&D Systems) at 50ng/ml, with supernatants containing soluble fractalkine at 50ng/ml, with 30$\mu$g/ml rabbit anti-CX3CR1 or rabbit IgG control (Interchim) for 30 minutes at 37°C in 5% CO2.

The supernatants containing soluble fractalkine were produced in RPMI basal medium by $3\times10^6$ HEK293 cells transfected with pCDNA 3.1(-) vector coding for soluble CX3CL1 or with control supernatants produced in RPMI basal medium by $3\times10^6$ HEK293 cells transfected with pCDNA 3.1(-) vector alone. The concentration of soluble fractalkine (CX3CL1) in the supernatants was assessed with an enzyme-linked immunosorbent assay (ELISA) detection kit (no. DY365 Duoset System for human CX3CL1/fractalkine: R&D Systems).

After incubation, medium containing effector cells was removed, target cells were washed with PBS with Ca2+ and Mg2+ and fluorescence was measured using multi-plate cell reader Cytofluor. Assays were performed in triplicate, and data were expressed as percent of lysis/control sample (sample treated in the same condition without effector cells).

The results are shown in Figures 7 and 8 (CMFDA assay of EPC cell lysis). Prior engagement of CX3CR1 activates NK cells lytic potential against TNF-$\alpha$ IFN-$\gamma$ stimulated EPC target cells (effector / target ratio : 15 : 1)

Our result shows that preincubation of the rabbit CX3CR1 antibody with purified NK cells does not block the fractalkine induced lytic effect of NK cells toward inflamed EPC but rather stimulates their cytotoxic activity towards endothelium.

This suggests that efficiency of CX3CR1 targeting may result in unexpected effects linked to various parameters linked to the agonist/ synergic effect of Fc mediated and antigen dependent of CX3CR1 antibodies and polymorphism dependent CX3CR1 fractalkine ligand recognition.

Better Knowledge of mechanisms that participate to the humoral and fractalkine dependent CX3CR1 engagement and consequent immune activation should help in the better design of therapeutic approaches targeting this pathway and allow better translation and monitoring of these therapies in clinical settings.

*Example 8: CX3CR1 expression on host unstimulted NK cells is correlated with host NK cell activation, graft function and anti HLA immunization status of transplanted host*

% CX3CR1 and CD16 expressing NK cell subsets (gated by flowcytometry within PBMC as CD3-CD56+) were analyzed in a cohort of 55 kidney transplanted patients evaluated at one year post graft. We show that distribution of these subsets was affected by the presence in the sera of transplant recipient of detectable Anti HLA antibodies detected by ELISA.

The results are shown in Figure 9.

Multivariate analysis of variables associated to CD16positive NK+ et CX3CR1 positive NK representation at one year post graft shows that it is influenced by presence of anti-HLA antibodies in the serum and also by creatinine clairance evaluation of renal function and immunosuppressive regimen.

| | % NK $CD16^+$ one year post graft | |
|---|---|---|
| variables | BCoef (SE) | *p* |

(continued)

| | % NK CD16+ one year post graft | |
|---|---|---|
| Hemodialysis duration | -0.08 (0.034) | 0.485 |
| Retarded graft function | -9.77 (4.46) | *0.01* |
| Therapeutic immunosuppressive regimen CsA/AZA | -9.32 (3.37) | *0.001* |
| Immunisation status prior graft | -9.51 (4.35) | *0.024* |
| Clairance at one year (ml/mn) | - | *0.035* |
| Adjusted $R^2$ | 0.305 | |

Interestingly lowered % CX3CR1 NK cells analyzed at one year post graft also appeared to be predictive of degradation of renal function evaluated by creatinine clairance at 3 years post graft

| | Creatinine Clairance 3 years post graft | |
|---|---|---|
| variables | BCoef (SE) | *p* |
| % CX3CR1 1 an de greffe | | 0.023 |
| Treatment | | 0.07 ns |
| Presence of anti HLA (ELISA) | | *0.012* |
| $R^2$ | 0.143 | |

*Example 9: % CX3CR1 and CD16 mfi expression are indicative of an activated humoral status of long term transplanted patients*

We analyzed % NK and CD3+CD8+ subsets expressing CX3CR1 in a cohort of 144 kidneys transplanted patients after a median period of 6 years post graft, and included in two randomized immunosuppressive regimen either (tacrolimus + mycophenolate mofetil) or (cyclosporine+ azathioprine). Transplanted patients were evaluated in reference values observed in 48 healthy donors.

We found that lower % CX3CR1 NK cells were observed in transplanted patients when compared to healthy donors (median, 25-75%percentile 79, 70-84). The % CX3CR1 NK cells were also observed to be lower in the patients treated with CSA/aza (median, 25-75%percentile 64.5, 48-72) as compared to those treated with TAC/MMF (68,59-79), p= 0.0153 suggesting a differential impact of treatment on CX3CR1 NK cells. Further more luminex assay of anti HLA antibodies in the serum of patients revealed that % CX3CR1 NK were lowered in transplanted patients with anti-HLA antibodies notably in the FK group with higher number of NK cells. This suggests that lowered distribution of the CX3CR1 NK cell subset might indicate the presence of an immunized status in these patients.

Interestingly in this group of transplanted patients, the % CX3CR1 NK cells was correlated to that of CD16 expressing cells (p<0.0001), to absolute counts of NK cells and to graft function evaluated by creatinine clairance at time of blood sampling (p= 0.036) but was unaffected by soluble serum CX3CR1 ligand/fractalkine levels.

The results are shown in Figure 10.

*Example 10: Analysis of phenotypic features of kidney Transplanted patients and Healthy Donors and rituximab induced modulation of CD16 and CD107a/LAMP-1 Activity*

The results are shown in Table 5.

*Example 11: highly variable capacity of patients to exert rituximab induced ADCCactivity can be evaluated by CD16 mfi in kidney transplanted patients*

Patients or healthy donor effector PBMC cells were incubated with rituximab therapeutic anti CD20 at concentrations of 10 μg/ml to test the ADCC effect towards autologous B cells, or with B-EBV transformed cell lines target cells expressing the CD20 antigen (GOE). We then observed the ADCC activation potential of NK cells mediated by CD16 engagement of the Fc fragment of rituximab and CD20 antigenic recognition at the surface of B lymphocyte target. Activation was evaluated through flow cytometry analysis of the induction of the granule exocytosis marker CD107a/LAMP-1 and by evaluation of the shift in CD16 expression indicative of rituximab Fc triggering (% and mfi of CD16).

| | Target BEBV GOE | RPMI +/-rituximab | Effector PBMC cells |
|---|---|---|---|
| Test | GOE B cells | Rituxim ab 10microg /ml | CD3-CD56+ NK cells within PBMC |
| Control 1 Analysis of rituximab induced ADCC towards autologous B cells | Autologou s B cells within PBMC | Rituxim ab 10microg /ml | CD3-CD56+ NK cells within PBMC |
| Control 2: natural cytotoxicity against B-EBV target | GOE B cells | RPMI medium | CD3-CD56+ NK cells within PBMC |
| Control 3 : reference activation and phenotypic features of NK cells in absence of Fc compound | | RPMI medium | CD3-CD56+ NK cells within PBMC |

Activation of granule exocytosis induced by rituximab evaluated against autologous B cells was evaluated by calculating the % CD107a/LAMP-1 CD3-CD56+ NK cells activated PBMC subset observed in presence of rituximab (control1) minus the activation observed in presence of sole RPMI medium (control 3)

The delta of CD107A/LAMP-1 granule exocytosis induced by rituximab observed between tube 1 and 3 was found to correlate with the number of B cells found within PBMC (p<0.0001), with the mfi of CD16 within CD3-CD56 cells (p= 0,0086) of patients in absence of stimulation. In the invitro ADCC test, the rituximab induced ADCC also correlated with the shift in CD16 mfi induced by rituximab toward autologous B cells (delta downregulation of CD16 mfi at the surface of CD3-CD56+ NK cells observed between tube 1 and 3, p<0.0001) or exogenous B-EBV cells (p< 0.0001) . The ADCC potential of patient to elicit an ADCC response against their B-EBV target were found to exibit high variability among transplanted patients and controls.

Interestingly, CD107A/LAMP-1 lamp analysis of effector cell activation induced by rituximab against B-EBV target cell lines or autologous B lymphocytes present in effector cell PBMC preparation was found to correlate to rituximab induced down regulation of CD16 mfi in CD3-CD56+ CD16+ NK cells in 167 individuals , but also to base line expression of CD16 in effector cells or combined index of % CX3CR1 and CD16 expression in NK cells from both transplanted (117 kidney transplanted patients) and 50 healthy control hosts analyzed . This suggests that levels of expression of CD16 are indicative of an individual's potential to mediate an antibody dependant NK cell activation.

| Lamp (B-EBV RITUX - BEBV) | Number of XY Pairs | Spearman r | P value (two-tailed) | P value summary |
|---|---|---|---|---|
| CD107A/LAMP-1 RPMI+Ritux - Lamp RPMI | 167 | 0,4271 | P<0.0001 | *** |
| MFI CD16 GOE-MFI CD16 GOE+Ritux | 167 | 0,4754 | P<0.0001 | *** |
| % NK CD16 | 165 | 0,2699 | 0,0005 | *** |
| MFI NK CD16 | 165 | 0,2662 | 0,0005 | *** |
| %CX3CR1+MfiCD16 in unstimulated cells | 163 | 0,2118 | 0,0066 | ** |

*Example 12: detection of CX3CR1 and CD16 polymporphisms at the transcript or genomic level*

12.1 Genomic analysis of FCR and CX3CR1 gene polymorphisms

*Multiplex SNaPshot reaction:*

A two-step method was developed for simultaneously analyze SNPs defining CX3CR1, FCRN, FcgR2A, FcgR3A, FcgR3B. The first step consisted of a multiplex PCR, by using primers flanking the SNPs to be characterized.

*Table 1 : Nucleotid positions*

| Gene | Allele 1 | Allele 2 | Nucleotide position | Amino-acid résidues |
|---|---|---|---|---|
| *CX3CR1* | *G-wt* | *A-mt* | 745/837 | V249I |
| *CX3CR1* | *C-wt* | *T-mt* | 849/931 | T280M |
| *FCRN* | *G-wt* | *A-m t* | 966 | K287R |
| *FCRN* | *6-wt* | *T-m* | 998 | L298V |
| *FcgR2A* | *A-wt* | *G-mt* | 468 | V139M |
| *FcgR2A* | *A-wt* | *G-mt* | 550 | R166H |
| *FcgR2A* | *C-wt* | *T-mt* | 665 | V204V |
| *FcgR3A* | *6-wt* | *A-m t* | 595 | D147G |
| *FcgR3A* | *T-wt* | *C-mt* | 627 | H158Y |
| *FcgR3A* | *C-wt* | *T-mt* | 653 | D166D |
| *FcgR3A* | *T-wt* | *G-mt* | 681 | F176V |
| *FcgR3A* | *T-wt* | *C-mt* | 763 | S203F |
| *FcgR3A* | *C-wt* | *T-mt* | 855 | R234stop |
| *FcgR3A* | *T-wt* | *C-mt* | 888 | L245F |
| *FcgR3B* | *A-wt* | *G-mt* | 227 | N65S |
| *FcgR3B* | *A-wt* | *G-mt* | 349 | V106I |
| *« wt = wild type» « mt = mutation » ; « nt = nucleotide position "* | | | | |

The multiplex PCR was developed to co-amplify 7 PCR fragments.

Multiplex PCR were performed respectively from 100 ng of genomic DNA in a final volume of 25 μL containing 1x PCR buffer, 1.5 mM $MgCl_2$, 0.2 mM of each dNTP, 0.1 unit of Taq DNA-polymerase and a defined concentration of each primer (table 2). All primers were designed using the «Primer 3» program (http://frodo.wi.mit.edu/cgi-bin/primer3/primer3_www.cgi). The « Integrated DNA technology » program (http://www.idtdna.com/analyzer/Applications/OligoAnalyzer) was used to test for potential primer-dimer conflicts and second hairpin structures.

Table 2

| Gene | Primers 5'-->3' sens (S) et reverse (R) | | Size of ampli fied | Concentration (μM) | | Vol |
|---|---|---|---|---|---|---|
| | | | | Initial | Final | for 25μL |
| *CX3CR 1* | S | CCGAGGTCCTCCAGGAAATC T (SEQ ID N°7) | 588 | 50 | 0.8 | 0.4 |
| | R | TCAGCATCAGGTTCAGGAA CTC (SEQ ID N°8) | | 50 | 0.8 | 0.4 |
| *FCRN* | S | AGTCAAAAGTGGCGATGAG C (SEQ ID N°9) | 826 | 50 | 0.8 | 0.4 |
| | R | CTGCCGTGAGTAGCAAGAC A (SEQ ID N°10) | | 50 | 0,8 | 0,4 |
| *FcGR2 A* | S | CCAGGAGGGAGAAACCATC (SEQ ID N°11) | 260 | 50 | 0,8 | 0,4 |
| | R | CTCTTCTCCCCTCCCTACAT (SEQ ID N°12) | | 50 | 0,8 | 0,4 |

(continued)

| Gene | Primers 5'-->3' sens (S) et reverse (R) | | Size of amplified | Concentration (μM) | | Vol |
|---|---|---|---|---|---|---|
| | | | | Initial | Final | for 25μL |
| *FcGR3 A* | S | ATATTTACAGAATGGCACA GG (SEQ ID N°13) | 168 | 50 | 0.8 | 0,4 |
| | R | ACGTGCTGAGCTTGAGTGA TGGTGATGTTCAC (SEQ ID N°14) | | 50 | 0,8 | 0,4 |
| *FcGR3 A* | S | TCCTAATAGGTTTGGCAGT G (SEQ ID N°15) | 250 | 50 | 0,8 | 0,4 |
| | R | AAATGTTCAGAGATGCTGC T (SEQ ID N°16) | | 50 | 0.8 | 0.4 |
| *FcGR3 B* | S | AAAGGCTGTGGTGTTCCTG (SEQ ID N°17) | 300 | 50 | 0.8 | 0.4 |
| | R | CCTTTGTTTCACCCTTTATT GG (SEQ ID N°18) | | 50 | 0.8 | 0.4 |

Amplification was carried out as follow, i.e., 95°C for 5 min for 1 cycle; 95°C for 15 sec, 59°C for 30 sec, 72°C for 90 sec for 30 cycles; 72°C for 7 min for 1 cycle.

After a control on 2% (w/v) agarose gel, 15 μl of PCR product was incubated with 5 units of Shrimp Alkaline Phosphatase and 1 units of Exonuclease-I (Euromedex; Souffelweyersheim, France) for 1 hour at 37°C followed by 15 min at 80°C to remove unincorporated primers and dNTPs.

The second step consisted of a multiplex extension primer reaction, i.e. an incorporation of a fluorescent ddNTP to extension primers annealed upstream or downstream to each SNP.

The reaction used the SNapShot kit (Applied Biosystems, Courtaboeuf, France) according to manufacturer's protocol in a final volume of 10 μL containing 3 μL of the PCR product, 5 μL of SNapShot mix and extension primers (for final concentration see table 3). Extension primers were designed to anneal immediately adjacent to the SNP site on either the sense or anti-sense DNA strand. Extension primer sequences were designed with different lengths by addition of a poly-T tail at their 5'-end, to allow their differentiation by capillary electrophoresis. Primers were synthesized by Eurogentec (Seraing, Belgium). Extension primers were purified by HPLC to remove incomplete synthesis products.

Table 3

| Gene (position) | Extension primers 5'→3' Sens (S)/ reverse (R) | | Concentration μM) | | Vol (μl) | Size (b) |
|---|---|---|---|---|---|---|
| | | | Initial | Final | | |
| *CX3CR1 (249)* | S | 5T-TCTTCTGGACACCCTACAAC (SEQ ID N°19) | 30 | 0.10 | 1 | 25 |
| *CX3CR1 (280)* | S | 10T-GGCCCTCAGTGTGACTGAG A (SEQ ID N°20) | 100 | 0.57 | 1.7 | 30 |
| *FCRN (287)* | S | 14T-GGGGCTGGCGCAGCCCCT CA (SEQ ID N°21) | 100 | 1.33 | 4 | 32 |
| *FCRN (298)* | S | 48T- AATCTCCAGCCAAGTCCTC C (SEQ ID N°22) | 100 | 0.33 | 1 | 68 |
| *FcgR2A (139)* | S | 35T-TCCAGGAGGGAGAAACCAT C (SEQ ID N°23) | 20 | 0.07 | 1 | 55 |
| *FcgR2A (166)* | S | 42T-AAAATCCCAGAAATTCTCCC (SEQ ID N°24) | 100 | 0.47 | 1.4 | 62 |
| *FcgR2A (204)* | S | 45T-AAGCCTGTGACCATCACTG T (SEQ ID N°25) | 100 | 0.30 | 0.9 | 65 |

(continued)

| Gene (position) | Extension primers 5'→3' Sens (S)/ reverse (R) | | Concentration μM) | | Vol (μl) | Size (b) |
|---|---|---|---|---|---|---|
| | | | Initial | Final | | |
| *FcgR3A (147)* | S | 20T-ATATTTACAGAATGGCAAAG (SEQ ID N°26) | 100 | 1.07 | 3.2 | 40 |
| *FcgR3A (158)* | S | 23T-TTCATCATAATTCTGACTTC (SEQ ID N°27) | 100 | 1.67 | 5 | 45 |
| *FcgR3A (166)* | S | 57T-CCAAAAGCCACACTCAAAG A (SEQ ID N°28) | 100 | 0.57 | 1.7 | 77 |
| *FcgR3A( 176)* | S | 30T-CCTACTTCTGCAGGGGGCT T (SEQ ID N°29) | 100 | 0.40 | 1.2 | 50 |
| *FcgR3A (203)* | R | 61T-TCAACCATCTCATCATTCT (SEQ ID N°30) | 100 | 1.07 | 3.2 | 80 |
| *FcgR3A (234)* | S | 38T-TCTCTGTGAAGACAAACATT (SEQ ID N°31) | 100 | 1.33 | 4 | 58 |
| *FcgR3A (245)* | S | 51T-GAGACTGGAAGGACCATAA A (SEQ ID N°32) | 100 | 1.33 | 4 | 71 |
| *FcgR3B (65)* | R | 54T-AGGCCTGGCTTGAGATGAG G (SEQ ID N°33) | 50 | 0.20 | 1.2 | 74 |
| *FcgR3B (106)* | S | 27T-CGGTGCAGCTAGAAGTCCA T (SEQ ID N°34) | 100 | 0.27 | 0.8 | 47 |

The reaction was carried as follows: 95°C for 10 seconds, 50°C for 5 seconds, 60°C for 30 seconds for 25 cycles.

The product of the extension reaction (10 μl) was purified with 1 unit of SAP for 1 hour at 37°C followed by 15 min at 80°C for enzyme inactivation.

Extension primers were designed to anneal immediately adjacent to the SNP site on either the sense or anti-sense DNA strand. Extension primer sequences were designed with different lengths by addition of a poly-T tail at their 5'-end, to allow their differentiation by capillary electrophoresis. Primers were synthesized by Eurogentec (Seraing, Belgium). Extension primers were purified by HPLC to remove incomplete synthesis products.

*Capillary electrophoresis conditions*

The fluorescence and size of extended products were determined by capillary electrophoresis on an ABI PRISM 3130XL genetic analyser (Applied Biosystem) using POP-7 polymer and 36-cm length capillary. Prior to loading onto the genetic analyzer, aliquots of purified SNapShot multiplex extension reaction (0.5 μl) were mixed with 9.25 μl of deionized formamide (Applied Biosystems) and 0.25 μL of size standard (GeneScan-120 LIZ ladder, Applied Biosystem). Capillary electrophoresis was performed according to the manufacturer's protocol.

*Data interpretation*

Data were analysed using GeneMapper v4.0 with specific detection parameters. Output files (.txt) exported by GeneMapper 4.0 were automatically formatted with an home-made script into files readable by "phenotype" of the Gene [rate] computer tools (13). This tool calculates the phenotype interpretation of any sets of submitted data.

*Method validation*

For each different alleles obtained, PCR products were sequenced on both strands using the BigDye Terminator v1.1 Sequencing Kit (Applied Biosystems) and analyzed on an automated fluorescence-based ABI PRISM 3130 XL Genetic analyser according to manufacturer's protocol.

CD16 generic primers

| | | | |
|---|---|---|---|
| CD16AB-328F | F | 3/5 | CCGGTGCAGCTAGAAGTCCA (SEQ ID N°35) |
| CD16AB-522R | R | 4/5 | TTCCAGCTGTGACACCTCAGG (SEQ ID N°36) |

CD16 primers detecting Polymorphic variants

| | | |
|---|---|---|
| CD16 F | F | |
| | | Ccggtgcagctagaagtccat (SEQ ID N°37) |
| CD16 158F R | R | Ctctgaagacacatttttactcccaaa (SEQ ID N°38) |
| CD16 158V R | R | Ctctgaagacacatttttactcccaac (SEQ ID N°39) |

CX3CR1 generic primers detecting both variants

| | | |
|---|---|---|
| CX3CR1-35 F | F | GGCTGACTGGCAGATCCAGAG (SEQ ID N°40) |
| CX3CR1-262 R | R | TGACACTCTTGGGCTTCTTGCT (SEQ ID N°41) |

Q RT-PCR of CD16 and CX3CR1 transcripts

Total RNA was isolated from PBMC cells or dissociated biopsy material using standard methods and RNeasy mini kits (QIAGEN Inc., Valencia, CA, USA) according to the manufacturer's instructions, including a DNase I digestion step to remove contaminating genomic DNA. After spectrophotometric quantification, 2 μg of total RNA was converted to cDNA by using 3 μl random primer hexamers (50 ng/μl, Amersham Biosciences) and 200 units of Supercript II reverse transcriptase (Invitrogen) in a reaction volume of 50 μl (10 min at 70°C, 2 min on ice, 60 min at 42°C, 5 min at 70°C) according to the manufacturer's instructions. Real-time PCR amplification was performed with the FastStart DNA Master$^{PLUS}$ SYBR Green I kit as recommended by the manufacturer, on a LightCycler instrument (Roche). The cycling conditions were 10 min at 95°C (hot-start PCR), followed by 40 cycles of 10 s at 95°C (denaturation), 10 s at 62°C (annealing) and 20 s at 72°C (elongation). Melting curve analysis was then performed to check the specificity of amplification. *CD16 and CX3CR1* expression were analyzed with primers specifically designed to identify all variants or polymorphic variants and a not exhaustive list of the primers used are described described in Table 1.

In relative quantification, reported values were expressed as the relative numbers of specific transcripts detected per $10^6$ *GAPDH* transcripts ($2^{-\Delta Ct}$ method). A calibrator sample, consisting of the mix of cDNA extracted from cryopreserved PBMC from two healthy donors expressing all gene varaints, was used to monitor inter-run variation. To allow absolute quantification, CD16 and CX3CR1 plasmids were linearised prior usage. In absolute quantification, the absolute copy number measured from plasmid standard curves was normalized by $10^4$ *GAPDH* copy number. All experiments were performed at least in duplicates.

Primers allowed characterisation of CX3CR1 and CD16 transcript levels in both PBMC and biopsies. While transcript levels may be indicative of gene polymorphism and CX3CR1 and CD16 expression levels, we showed that rituximab induced down modulation of CD16 and CX3CR1 cell surface phenotype during the 4 hour CHAT assay did not traduce in associated modification of CD16 and CX3CR1 transcript levels as analyzed in PBMC by Q-RT PCR. CX3CR1 transcript levels were analyzed in 64 biopsies from cardiac transplanted patients and show high degree of variability that may characterize the graft immune infiltrate. Relative values expressed as the relative numbers of specific transcripts detected per $10^6$ *GAPDH* transcripts ($2^{-\Delta Ct}$ method) varied from 25 to 9685 among biopsies examined with a median value observed of 1287.

## Claims

**1.** An *in vitro* method for determining a subject cellular humoral activity, which comprises the step of:

(ii) determining the expression profile of at least the CX3CR1 gene in effector cells expressing FCRG and CX3CR1, preferably $CD3^-$ $CD56^+$ NK cells
(iii) comparing said expression profile to at least one control such as control values of FCRG and CX3CR1 expressing subsets in representative healthy donor control cohorts.

**2.** The method of claim 1 for determining a subject cellular humoral activity directed against a specific antigen which comprises the step of:

(i) incubating :

- effector cells corresponding to FCRG and CX3CR1 expressing cells, preferably NK cells;
- Serum, or any medium containing therapeutic Fc compound or compounds interfering with the fractalkine CX3CR1 pathways, preferably a serum from said subject; and
- a target antigen preferentially expressed at the surface of a cell;

(ii) determining the expression profile of at least the CX3CR1 in said effector cells, preferably by flow cytometry analysis; and
(iii) comparing said expression profile to at least one control.

**3.** The method of claim 1 and 2, wherein said method comprises the step of determining the expression profile of at least the CX3CR1 gene and of at least one FCRG gene, preferentially said FCRG gene is CD16 gene.

**4.** The method of claim 1, 2 or 3, wherein said method further comprises the step of determining the lysis of the cell expressing said target antigen at its surface, such as by analysing target cell derived microparticule profile resulting from cellular humoral injury of target cells and comparing said lysis to at least one control.

**5.** The method of any of claims 1, 2, 3 or 4, wherein the expression profile-determining step (ii) of said method is assessed by analyzing the expression profile of the protein(s) translated from said gene(s).

**6.** The method of claim 5, wherein the expression profile-determining step (ii) of said method of the invention is assessed by analysing the cell surface expression profile of the protein(s) translated from said gene(s), preferably by Flow cytometry.

**7.** The method of any one of claims 1 to 6, wherein said method further comprises the step (v) of determining whether an expression level of said gene(s) is(are) increased or decreased relative to the control; wherein a decreased expression level of said gene(s) is indicative of an increased cellular humoral activity directed against said antigen of said subject.

**8.** The method of any one of claims 1 to 7, wherein it can be used in the following applications: Transplantation (including cross match incompatibility, recipient monitoring, graft function, acute and chronic rejection, vascular disorders), transfusion (including Cross match incompatibility Platelet transfusion inefficiency, refractory response to platelet transfusion therapy, Transfusion Related Acute Lung Injury (TRALI), LBP (Labile blood product) selection in transfusion settings, Post transfusional shock incident Feto-maternal incompatibility Newborn low platelets), diagnostic and prevention in reproductive medicine (including implantation failure, Infertility issues with etiology of auto antibodies, vascular disorders, foetal growth retardation, preeclampsia), autoimmune disease: diagnostic prognostic and follow-up, in particular as example test of autoantibodies driven vasculopathy, PTT purpura, chronic and acute immune thrombocytopenic purpura, Anti-phospholipid syndrom, lupus, vascular inflammatory diseases, Neurological diseases, monitoring of HIV infection and tretament, host related therapeutic efficiency, for example in auto and alloimmune processes, cancer and viral diseases, therapeutic monotoring, design of novel therapeutic protocols aiming at limiting humoral deleterious effects of allo or auto antibodies, evaluation of novel anti cancer or anti viral therapies using antibody or Fc based compounds agonist or antagonist to specifically eliminate tumor cells expressing specific antigens, Vaccination protocoles, Indication of vaccination efficacy, Design of vaccination protocol's evaluation, monitoring of Arthrosclerosis, HIV or viral infection susceptibility or evolution, Viral serology evaluation, and Cellular and Tissular regenerative medicine.

**9.** The method of any one of claims 1 to 8, wherein:

(i) said subject is a subject that has to be transplanted and the method of the invention is for determining the risk of graft dysfunction or alloantibodies mediated graft rejection of said subject;
(ii) said subject is a transplanted subject and the method of the invention is for determining the risk of chronic or acute graft rejection, graft dysfunction or cardiovascular complications resulting from the immunization status of said sub ject;
(iii) said subject is a transfused subject and the method of the invention is for determining the risk of transfusion reaction in said subject because of antibodies;
(iv) said subject has to be transfused and the method of the invention is for determining the risk of transfusion reaction in said subject because of antibodies
(v) said subject is a subject suffering from a auto-immune disease and the method of the invention is for determining the risk of evolution of said subject auto-immune disease because of autoantibodies;
(vi) said subject is to be transfused and the method of the invention is for determining the infused blood product in said subject
(vii) said subject is a female at risk for implantation failure and the method of the invention is for determining the risk of fetus rejection for said pregnant female because of auto or alloantibodies.
(viii) Said subject is a women experiencing immune related disorders in the reproductive field (implantation failure, miscarriage, vascular disorders, foetal growth retardation, preclampsia) the method of the invention is for determining the risk of reproductive failure for said pregnant female because of auto or alloantibodies.
(ix) Said subject is submitted to treatment aiming to reduce humoral activity (Intravenous immunoglobulins, plasma exchange, anti B lymphocyte therapy, immunosuppressive regimen, therapeutic antibodies, agonist or antagonist therapy targeting FcR or CX3CR1 pathways) and the method of invention is to determine the potential efficiency before treatment and/or to monitor the efficiency of the treatment in said subject; and/or
(x) Said subject is submitted to a treatment based on agonist or antagonist therapy targeting FcR, IgG or CX3CR1 pathways to limit any disease including cardiovascular, autoimmune diseases, neurologic diseases, cancer, viral infection and said method is to refine indication and efficiency of a treatment in said subject
(xi) Said subject is submitted to vaccination therapy or therapy using exosome or microparticles expressing target antigens as vehicles of functional activities that modulate fractalkine-CX3CR1 and Fc-FcR pathways.

**10.** A kit for determining a subject cellular humoral activity comprising (i) at least one mean for determining the expression profile of CX3CR1 gene in effector cells expressing FCRG and CX3CR1, preferably in lymphocytes, $CD3^{-}CD56^{+}$ NK cells and monocytes.

**11.** The kit of claim 10, wherein said kit further comprises (ii) at least one mean for determining the expression profile of CD16 gene in effector cells expressing FCRG and CX3CR1, preferably in $CD3^{-}CD56^{+}$ NK cells.

**12.** Use, for determining a subject cellular humoral activity directed against an antigen, of the kit as defined in any one of claims 10 to 11.

TABLE 6

| **Effector cells** | **Serum or Fc antiCD20** | **HLA-A2Targets cells** | % CX3CR1 | % CD107 | MfiCD16 pos | % CD16dim | |
|---|---|---|---|---|---|---|---|
| PBMC TRI | ACAL-SERUM GAU | none | 78,1 | 12,27 | 73 | 13,27 | CTL1 |
| PBMC TRI | PBMC autologous ACAL-Serum TRI | none | 76,73 | 14,7 | 63,1 | 15,93 | CTL2 |
| PBMC TRI | PBMC Autologous serum TRI + Ritux 10 | none | 62,27 | 27,93 | 29,5 | 45,17 | TEST1 RITUX auto B |
| PBMC TRI | Autologous serum TRI + Ritux 10 | B-EBV HLA-A2 | 40,6 | 30,3 | 5,45 | 57,83 | EST2 RITUX B-EBV transformed |
| PBMC TRI | Patient Serum ACAL + anti HLA-A2 | none | 61,5 | 12,57 | 17,6 | 57,67 | CTL3 |
| PBMC TRI | Patient Serum ACAL + anti HLA-A2 | B-EBV A2 | 35,03 | 29,07 | 8,32 | 51,27 | TEST3 ANTI HLA-A2 serum |
| PBMC TRI | Serum autologue TRI | B-EBV A2 | 70,57 | 12,07 | 52,1 | 21,67 | CTL4 |
| PBMC TRI | ACAL-SERUM GAU | B-EBV A2 | 78,43 | 7,9 | 65,7 | 15,27 | TEST 4 ACAL negative serum |
| | | | | | | | |
| Degranulating activity | CD107 ritux against autologous B lymphocyte | PBMC | | | TEST1-CTL2 | | 13,23 |
| | CD107 Ritux againt B EBV transformed Cell | lines | | | TEST2-CTL4 | | 18,23 |
| | CD107 HLA2 SERUM | | | | TEST3-CTL4 | | 17 |
| | CD107 HLA2 SERUM | | | | TEST4-CTL1 | | -4,37 |

(continued)

| **Effector cells** | **Serum or Fc antiCD20** | **HLA-A2Targets cells** | % CX3CR1 | % CD107 | MfiCD16 pos | % CD16dim | |
|---|---|---|---|---|---|---|---|
| HHCA SCORE | | | | | | | |
| DELTA% CX3CR1+ DELTAMfi CD16D16 ritux against autologous B lymphocyte PBMC | | | RITUX Bly | | CTL2-TEST 1 | | 48,06 |
| | Ritux againt B EBV transformed Cell lines | | Ritux B-LCL | | CTL4-TEST 2 | | 76,62 |
| | Evaluation of HLA2 SERUM activity | | HLA-A2 serum | | CTL4-TEST 3 | | 79,32 |
| | Evaluation of HLA-negative serum activity | | HLA negative serum | | CTL1-TEST 4 | | -7,63 |

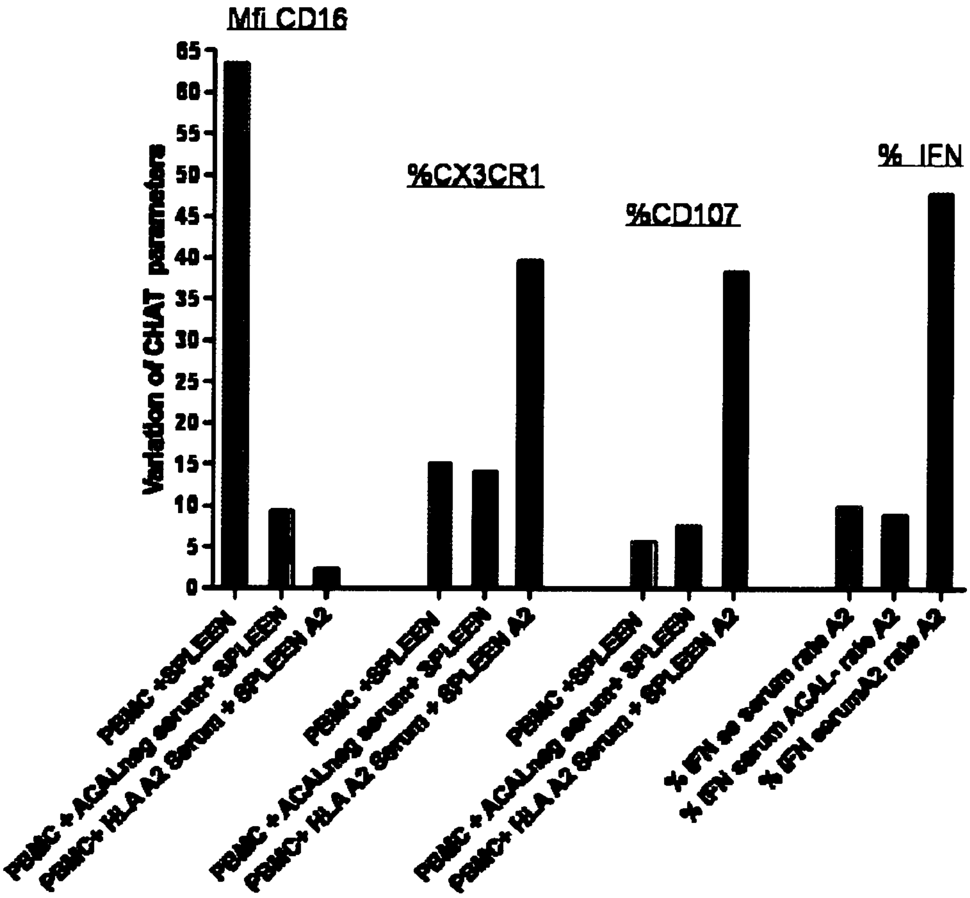
Mfi CD16
%CX3CR1
%CD107
% IFN
Variation of CHAT parameters
0
5
10
15
20
25
30
35
40
45
50
55
60
65
PBMC +SPLEEN
PBMC + ACALneg serum+ SPLEEN
PBMC+ HLA A2 Serum + SPLEEN A2
PBMC +SPLEEN
PBMC + ACALneg serum+ SPLEEN
PBMC+ HLA A2 Serum + SPLEEN A2
PBMC +SPLEEN
PBMC + ACALneg serum+ SPLEEN
PBMC+ HLA A2 Serum + SPLEEN A2
% IFN serum ACAL- rate A2
% IFN serumA2 rate A2

FIGURE 1

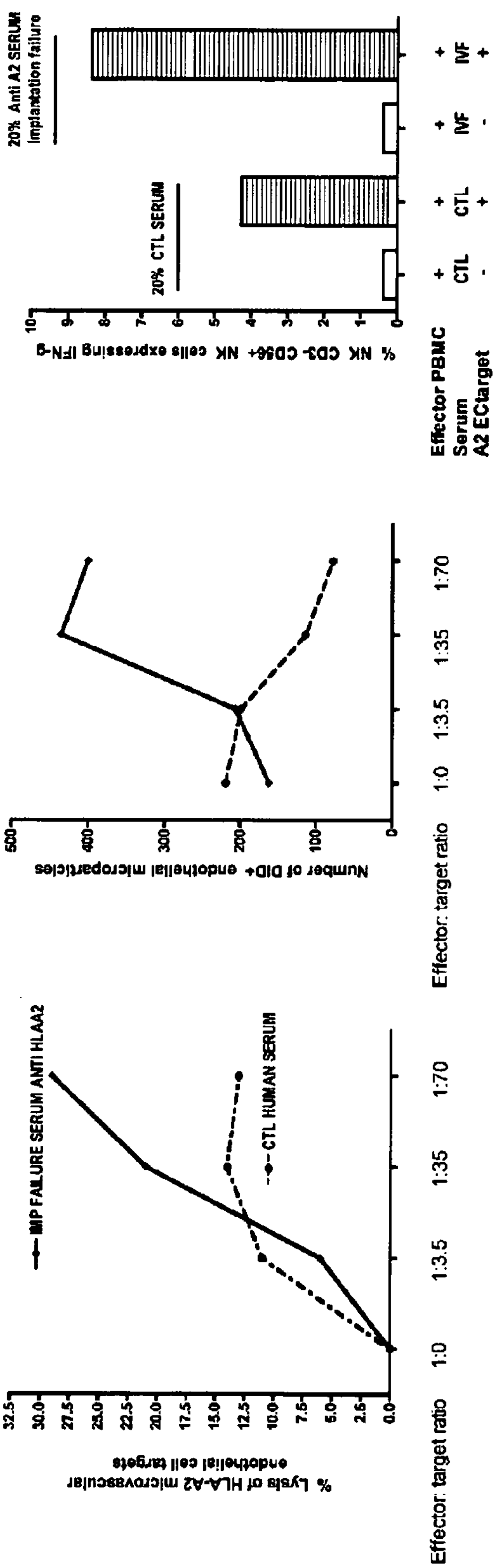

% Lysis of HLA-A2 microvascular endothelial cell targets
IMP FAILURE SERUM ANTI HLAA2
CTL HUMAN SERUM
Effector: target ratio
1:0
1:3.5
1:35
1:70
Number of DID+ endothelial microparticles
% NK CD3- CD56+ NK cells expressing IFN-g
20% CTL SERUM
20% Anti A2 SERUM Implantation failure
Effector PBMC
Serum
A2 ECtarget
CTL
IVF


FIGURE 2

## Flow cytometry detection of anti endothelial binding activity in serum or intravenous Immunoglobulin

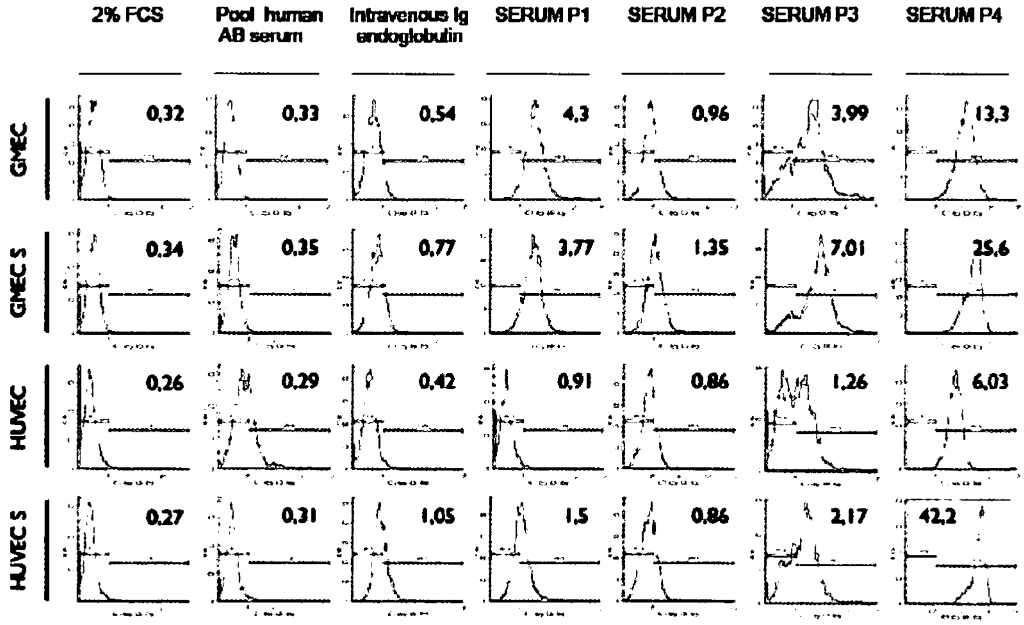


FIGURE 3

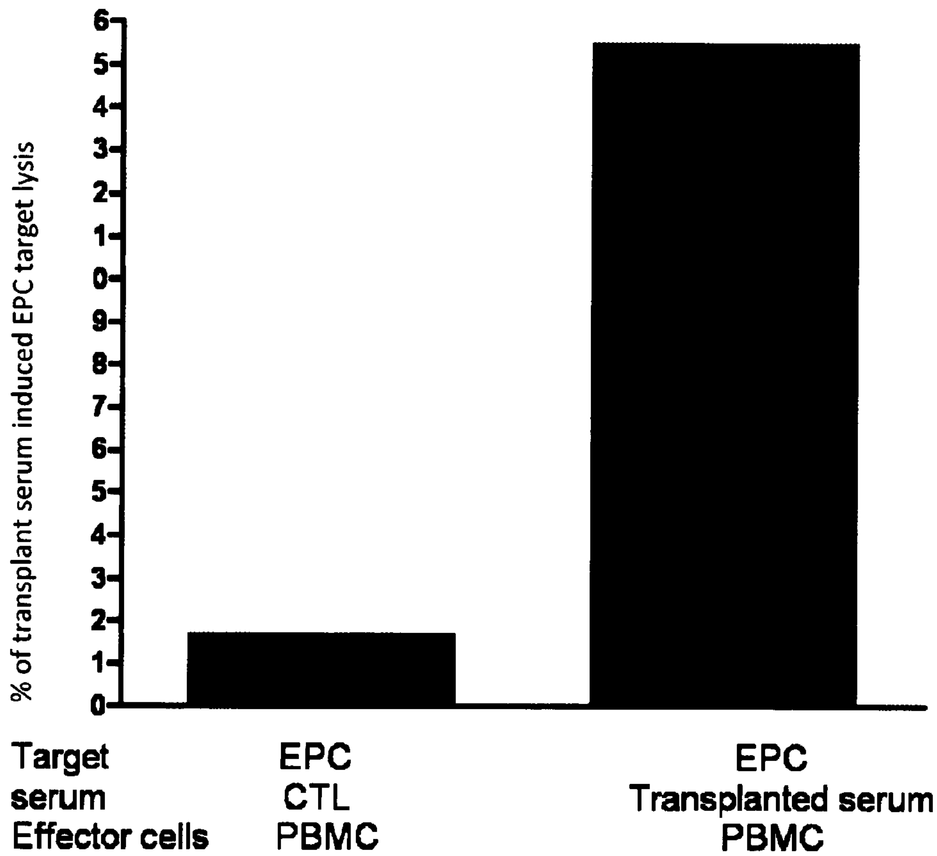
% of transplant serum induced EPC target lysis
Target
serum
Effector cells
EPC
CTL
PBMC
EPC
Transplanted serum
PBMC

FIGURE 4

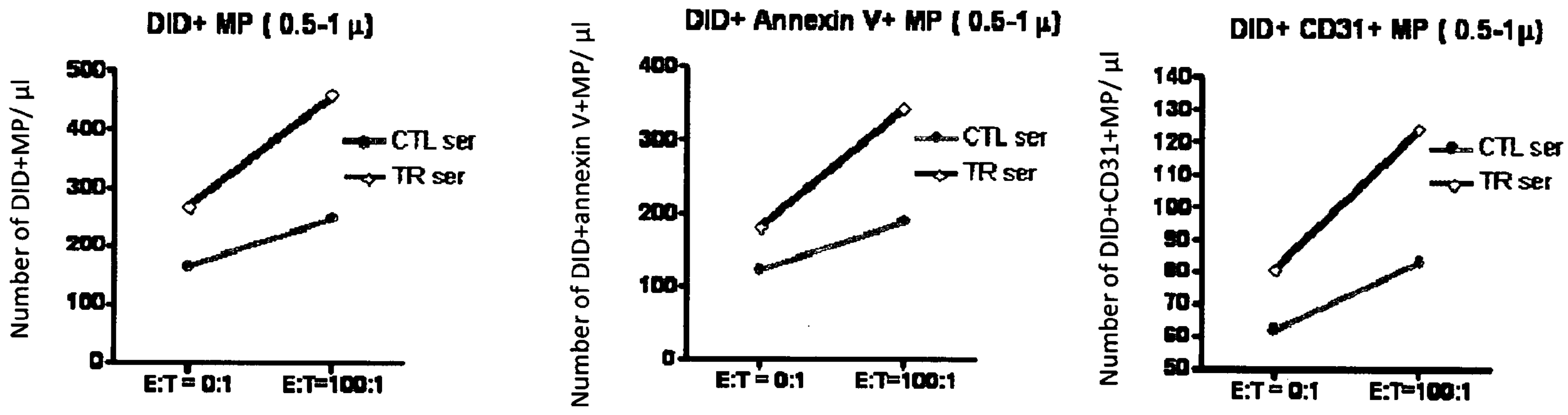
DID+ MP ( 0.5-1 µ)
Number of DID+MP/ µl
500
400
300
200
100
0
E:T = 0:1
E:T=100:1
CTL ser
TR ser
DID+ Annexin V+ MP ( 0.5-1 µ)
Number of DID+annexin V+MP/ µl
400
300
200
100
0
E:T = 0:1
E:T=100:1
CTL ser
TR ser
DID+ CD31+ MP ( 0.5-1µ)
Number of DID+CD31+MP/ µl
140
130
120
110
100
90
80
70
60
50
E:T = 0:1
E:T=100:1
CTL ser
TR ser

FIGURE 5

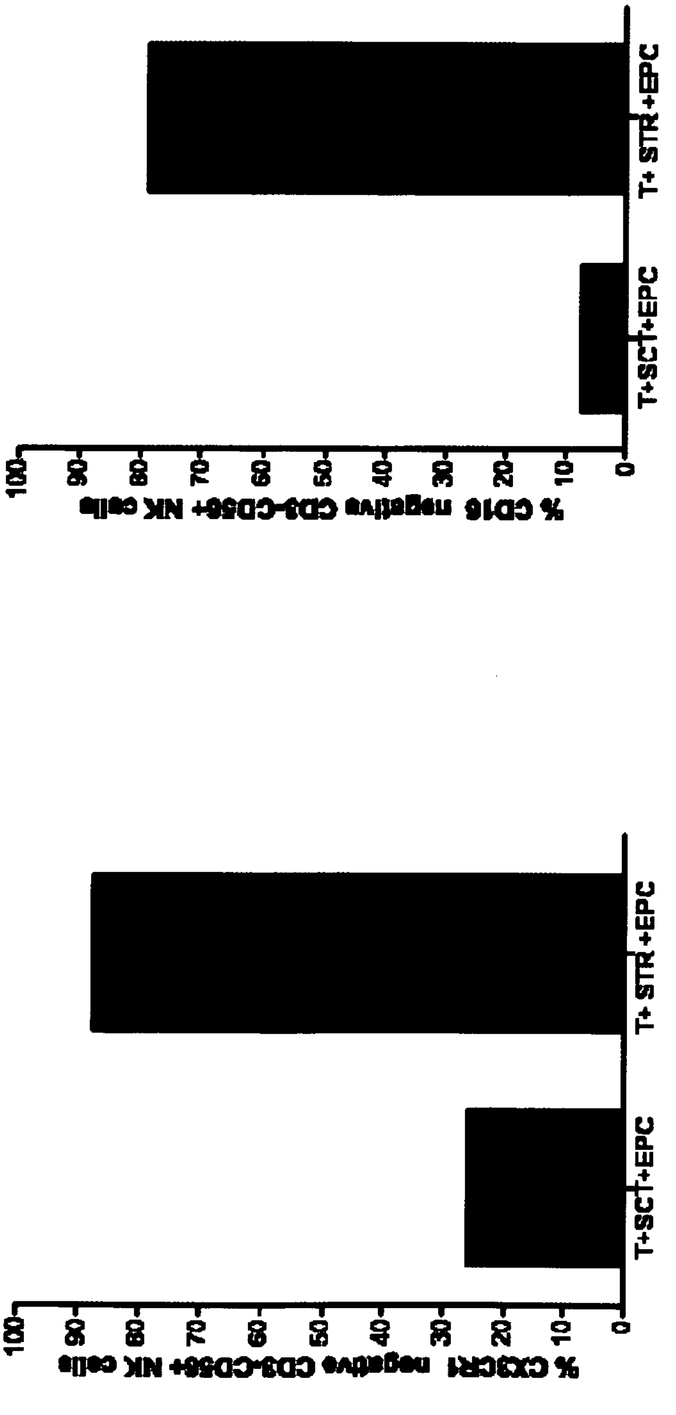
% CD16 negative CD3-CD56+ NK cells
100
90
80
70
60
50
40
30
20
10
0
T+SCT+EPC
T+ STR +EPC
% CX3CR1 negative CD3-CD56+ NK cells
T+SCT+EPC
T+ STR +EPC

FIGURE 6

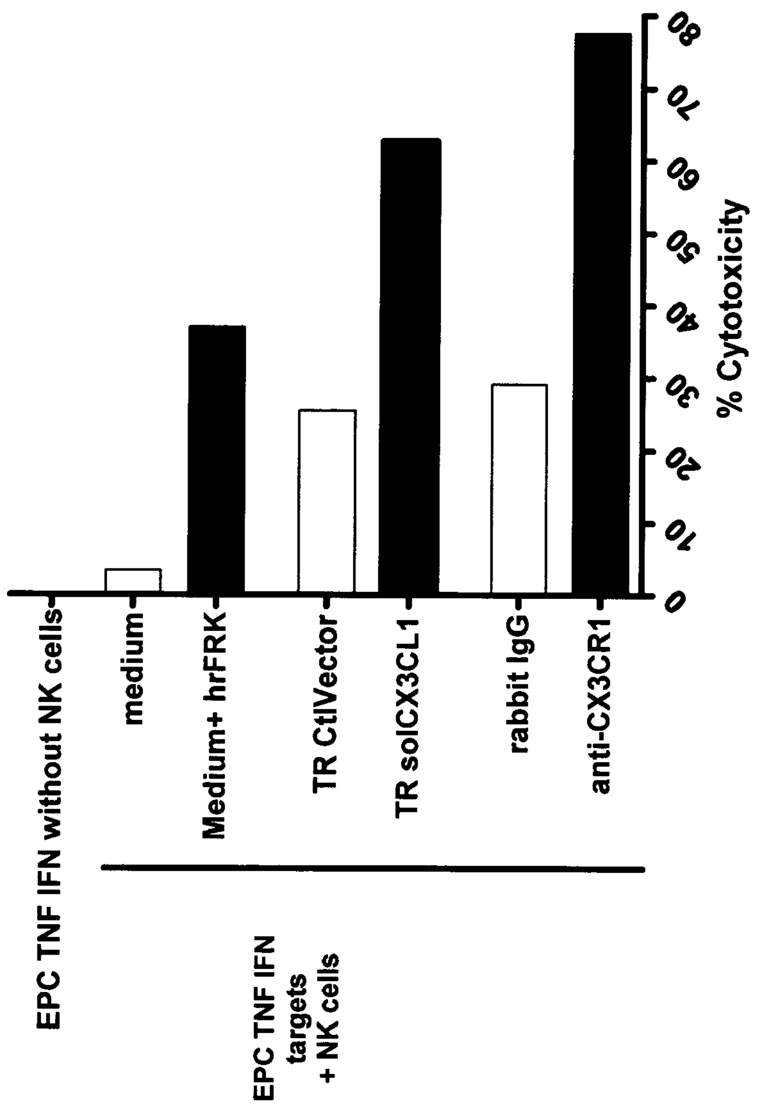
EPC TNF IFN without NK cells
medium
Medium+ hrFRK
TR CtlVector
TR solCX3CL1
rabbit IgG
anti-CX3CR1
EPC TNF IFN targets + NK cells
% Cytotoxicity
0
10
20
30
40
50
60
70
80

FIGURE 7

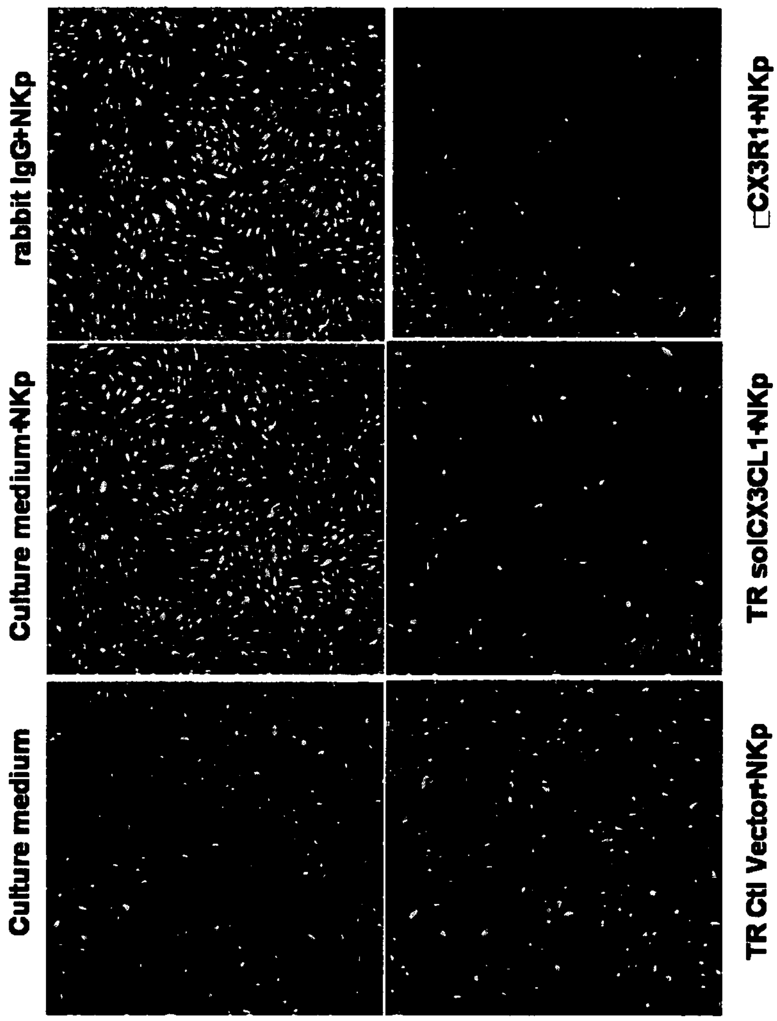
Culture medium
Culture medium+NKp
rabbit IgG+NKp
TR Ctl Vector+NKp
TR solCX3CL1+NKp
□CX3R1+NKp

FIGURE 8

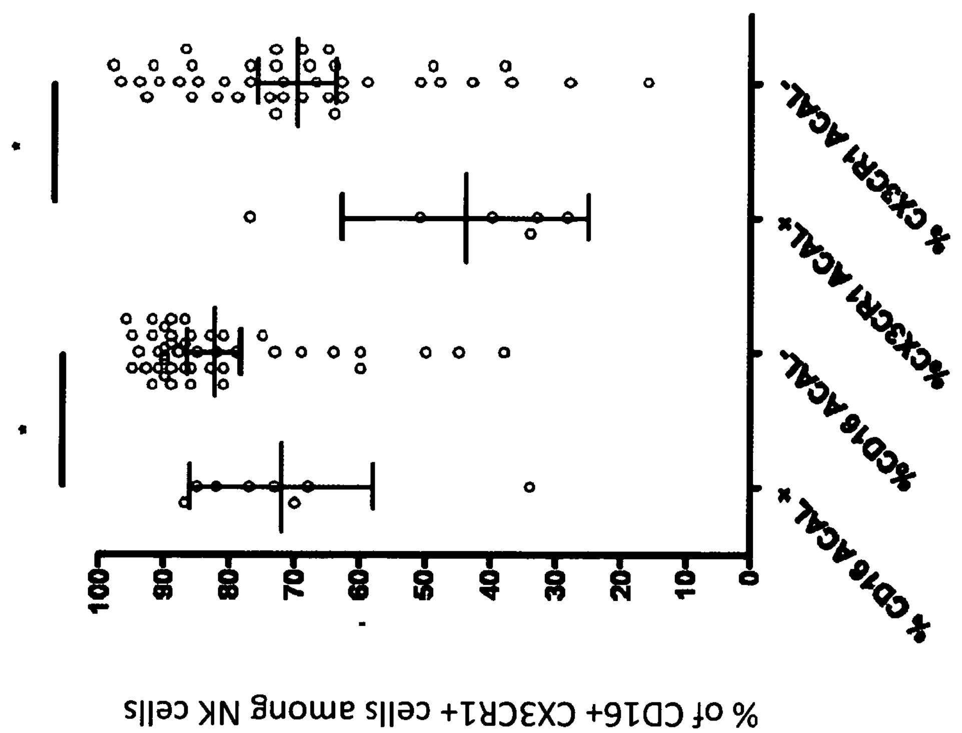
% of CD16+ CX3CR1+ cells among NK cells
100
90
80
70
60
50
40
30
20
10
0
% CD16 ACAL+
%CD16 ACAL-
%CX3CR1 ACAL+
% CX3CR1 ACAL-
*
*

FIGURE 9

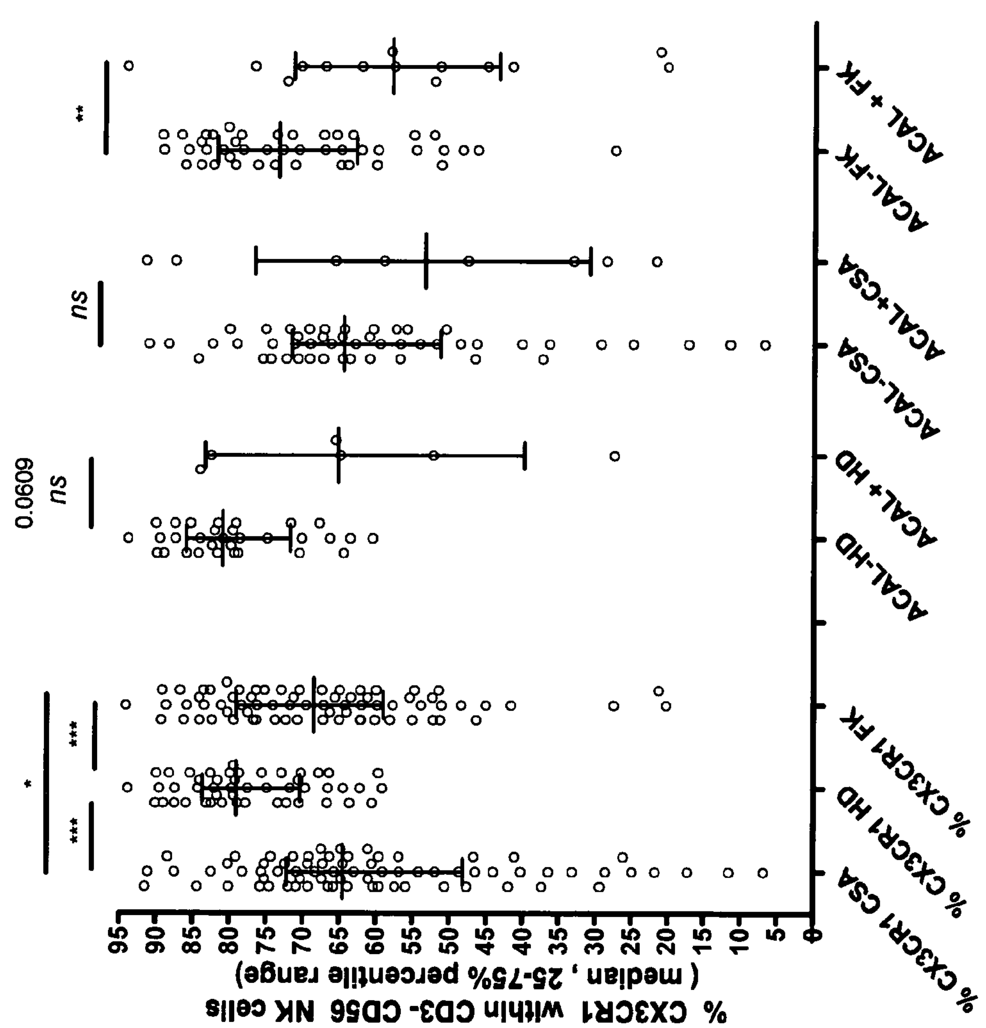

% CX3CR1 within CD3- CD56 NK cells
( median , 25-75% percentile range)
95
90
85
80
75
70
65
60
55
50
45
40
35
30
25
20
15
10
5
0
% CX3CR1 CSA
% CX3CR1 HD
% CX3CR1 FK
ACAL-HD
ACAL+ HD
ACAL-CSA
ACAL+CSA
ACAL-FK
ACAL + FK
***
*
***
0.0609
ns
ns
**


FIGURE 10

| Healthy Donor control values | Number of values | Minimum | 25% Percentile | Median | 75% Percentile | Maximum | Mean | Std. Deviation | Std. Error | Passed normality test (alpha=0.05)? |
|---|---|---|---|---|---|---|---|---|---|---|
| LAMP RPMI+Ritux - Lamp RPMI | 51 | -5,87 | 0,46 | 2,29 | 5,43 | 10,61 | 2,972 | 3,573 | 0,5003 | Yes |
| LAMP GOE+Ritux - Lamp GOE | 51 | 8,78 | 18,28 | 24,27 | 32,83 | 42,97 | 25,56 | 8,778 | 1,229 | Yes |
| MFI CD16 RPMI - MFI CD16 RPMI+Ritux | 51 | -9 | 11,7 | 26,1 | 46 | 92,9 | 29,91 | 23,71 | 3,321 | Yes |
| MFI CD16 GOE - MFI CD16 GOE+Ritux | 51 | 55,12 | 97,1 | 116,1 | 132,3 | 203,8 | 116,2 | 30,31 | 4,244 | Yes |
| %CX3CR1+MfiCD16 | 48 | 71,55 | 112,7 | 126,3 | 134,1 | 155,7 | 123,6 | 17,05 | 2,461 | Yes |
| %NK CD16 | 50 | 54,61 | 85,93 | 90,09 | 92,57 | 96,31 | 88,37 | 6,734 | 0,9524 | No |
| MFI NK CD16 | 50 | 26,2 | 40,95 | 48,4 | 56,8 | 69,3 | 48,41 | 10,94 | 1,547 | Yes |
| %NK CX3CR1 | 48 | 27,45 | 67,28 | 79,04 | 84,13 | 93,88 | 75,53 | 12,13 | 1,752 | No |
| %CX3CR1 8 | 48 | 1,98 | 6,085 | 13,26 | 21,9 | 79,84 | 18,78 | 18,34 | 2,648 | No |

| Transplanted CSA + Aza | Number of values | Minimum | 25% Percentile | Median | 75% Percentile | Maximum | Mean | Std. Deviation | Std. Error | |
|---|---|---|---|---|---|---|---|---|---|---|
| LAMP RPMI+Ritux - Lamp RPMI | 55 | -2,27 | -1,03 | 0,08 | 1,13 | 10,87 | 0,5687 | 2,48 | 0,3344 | No |
| LAMP GOE+Ritux - Lamp GOE | 55 | 3,45 | 9,75 | 12,62 | 22,57 | 39,73 | 15,78 | 8,834 | 1,191 | No |
| MFI CD16 RPMI - MFI CD16 RPMI+Ritux | 55 | -21 | 1,5 | 9,4 | 22 | 82 | 13,72 | 18,21 | 2,455 | No |
| MFI CD16 GOE - MFI CD16 GOE+Ritux | 55 | 25,4 | 61,7 | 83,9 | 98,7 | 157,9 | 82,21 | 28,35 | 3,823 | Yes |
| %CX3CR1+MfiCD16 | 73 | 28,65 | 78,08 | 100,1 | 113,4 | 143 | 96,31 | 26,12 | 3,058 | Yes |
| %NK CD16 | 74 | 32,7 | 64,31 | 78,13 | 87,43 | 96,86 | 74,19 | 16,13 | 1,876 | No |
| MFI NK CD16 | 74 | 11,3 | 28,15 | 36,65 | 45,55 | 64,1 | 36,65 | 11,52 | 1,339 | Yes |
| %NK CX3CR1 | 73 | 6,82 | 48,14 | 64,51 | 72,18 | 91,43 | 59,67 | 18,98 | 2,221 | No |
| %CX3CR1 8 | 77 | 0,5 | 9,255 | 23,04 | 38,53 | 72,1 | 24,55 | 17,7 | 2,017 | Yes |

| Transplanted FK+MMF CATM2 | Number of values | Minimum | 25% Percentile | Median | 75% Percentile | Maximum | Mean | Std. Deviation | Std. Error | Passed normality test (alpha=0.05) |
|---|---|---|---|---|---|---|---|---|---|---|
| LAMP RPMI+Ritux - Lamp RPMI | 61 | -5,82 | -0,17 | 1,08 | 2,43 | 13,89 | 1,452 | 3,01 | 0,3854 | No |
| LAMP GOE+Ritux - Lamp GOE | 61 | -0,26 | 14,34 | 20,79 | 28,32 | 47,56 | 21,47 | 10,16 | 1,3 | Yes |
| MFI CD16 RPMI - MFI CD16 RPMI+Ritux | 61 | -41 | 2,15 | 12,8 | 21,55 | 48 | 11,78 | 17,06 | 2,184 | Yes |
| MFI CD16 GOE - MFI CD16 GOE+Ritux | 61 | 1 | 66,93 | 93,19 | 115,6 | 205,3 | 92,8 | 36,97 | 4,734 | Yes |
| %CX3CR1+MfiCD16 | 71 | 32,36 | 93,32 | 108 | 122,2 | 150,9 | 106,1 | 22,53 | 2,674 | No |
| %NK CD16 | 71 | 49,81 | 78,89 | 86,67 | 90,58 | 96,34 | 83,76 | 9,951 | 1,181 | No |
| MFI NK CD16 | 71 | 12,1 | 31 | 39,4 | 46,8 | 62,3 | 38,95 | 11,16 | 1,324 | Yes |
| %NK CX3CR1 | 71 | 20,26 | 58,15 | 67,37 | 79,39 | 94,11 | 67,19 | 15,48 | 1,838 | No |
| %CX3CR1 8 | 69 | 1,55 | 10,25 | 20,6 | 33,71 | 68,1 | 24,44 | 17,28 | 2,08 | No |

TABLE 5

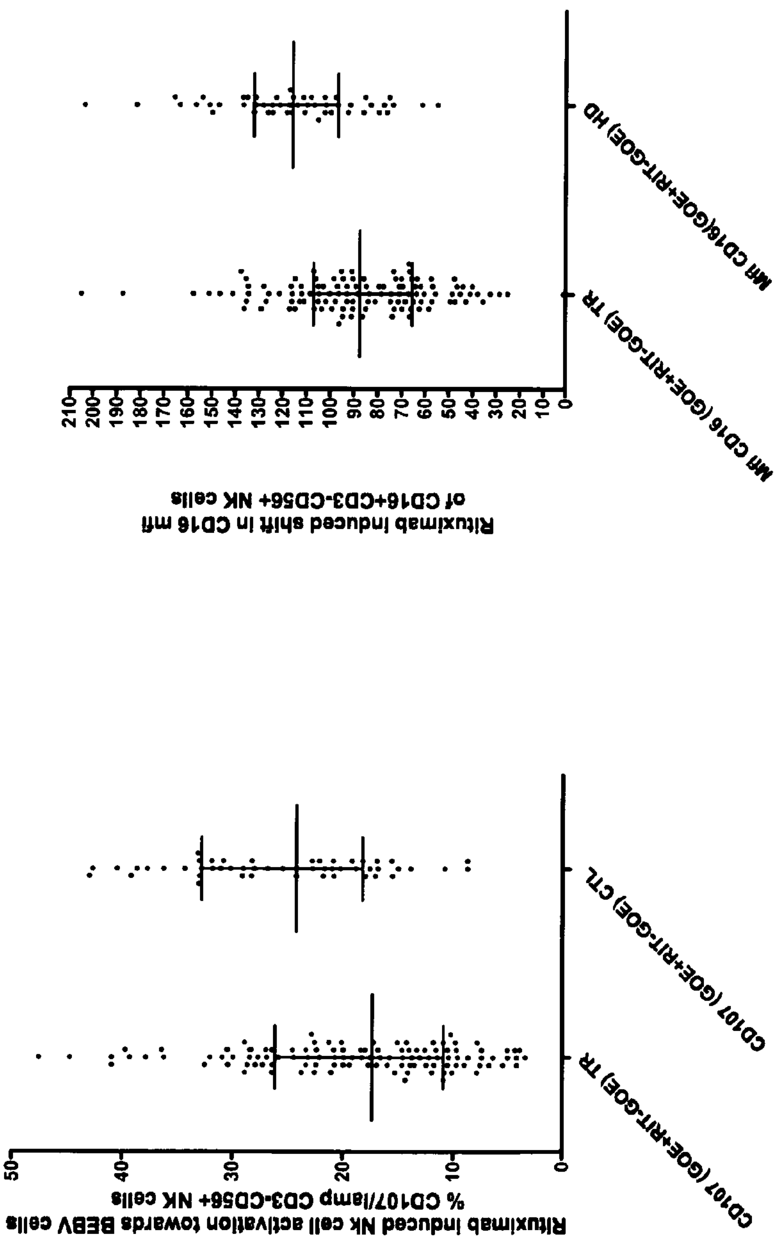
Rituximab induced shift in CD16 mfi
of CD16+CD3-CD56+ NK cells
Mfi CD16 (GOE+RIT-GOE) TR
Mfi CD16(GOE+RIT-GOE) HD
Rituximab induced NK cell activation towards BEBV cells
% CD107/lamp CD3-CD56+ NK cells
CD107 (GOE+RIT-GOE) TR
CD107 (GOE+RIT-GOE) CTL

FIGURE 11

Europäisches Patentamt
European Patent Office
Office européen des brevets

# EUROPEAN SEARCH REPORT

Application Number
EP 10 35 8003

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VACHER-COPONAT HENRI ET AL: "Natural killer cell alterations correlate with loss of renal function and dialysis duration in uraemic patients." NEPHROLOGY, DIALYSIS, TRANSPLANTATION : OFFICIAL PUBLICATION OF THE EUROPEAN DIALYSIS AND TRANSPLANT ASSOCIATION - EUROPEAN RENAL ASSOCIATION APR 2008 LNKD- PUBMED:18029366, vol. 23, no. 4, April 2008 (2008-04), pages 1406-1414, XP002605894 ISSN: 1460-2385<br>* page 1406, column 2, lines 18-28 *<br>* page 1407, column 1, lines 1-5 *<br>* page 1407, column 2, line 34 *<br>* page 1407, column 2, line 37 *<br>* page 1407, column 2, line 56 - page 1408, column 1, line 14 *<br>* page 1409, column 2, lines 23-27 *<br>* page 1409, column 2, lines 57-61; figure 3; table 4 *<br>-----<br>-/-- | 1-12 | INV.<br>G01N33/50<br>G01N33/564<br>G01N33/566 |

| TECHNICAL FIELDS SEARCHED (IPC) |
|---|
| G01N |

The present search report has been drawn up for all claims

1

| Place of search | Date of completion of the search | Examiner |
|---|---|---|
| Munich | 20 October 2010 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 35 8003

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | UMEHARA H ET AL: "Fractalkine and vascular injury" TRENDS IN IMMUNOLOGY, ELSEVIER, RAHWAY, NJ, US LNKD- DOI:10.1016/S1471-4906(01)02051-8, vol. 22, no. 11, 1 November 2001 (2001-11-01), pages 602-607, XP004319768 ISSN: 1471-4906 * abstract * * page 602, column 2, lines 33-38 * * page 603, column 2, line 55 - page 604, column 1, line 3 * * page 604, column 1, lines 31-38 * * page 604, column 2, lines 1-4 * * page 604, column 2, lines 10-22 * * page 605, column 1, lines 51-55 * * page 606, column 1, lines 10-18 * * page 606, column 2, lines 48-52 * ----- | 1-12 | |
| Y | HISANORI UMEHARA ET AL: "Fractalkine in rheumatoid arthritis and allied conditions" MODERN RHEUMATOLOGY ; OFFICIAL JOURNAL OF THE JAPAN COLLEGE OF RHEUMATOLOGY, SPRINGER-VERLAG, TO LNKD- DOI:10.1007/S10165-006-0471-9, vol. 16, no. 3, 1 June 2006 (2006-06-01), pages 124-130, XP019387365 ISSN: 1439-7609 * the whole document * ----- -/-- | 1-12 | TECHNICAL FIELDS SEARCHED (IPC) |

1

The present search report has been drawn up for all claims

| Place of search | Date of completion of the search | Examiner |
|---|---|---|
| Munich | 20 October 2010 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 35 8003

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CORNELL L D ET AL: "Kidney transplantation: Mechanisms of rejection and acceptance" ANNUAL REVIEW OF PATHOLOGY: MECHANISMS OF DISEASE, ANNUAL REVIEWS, US LNKD- DOI:10.1146/ANNUREV.PATHMECHDIS.3.121806.151508, vol. 3, 1 February 2008 (2008-02-01), pages 189-220, XP008123514 ISSN: 1553-4006 [retrieved on 2007-09-12] * the whole document * ----- | 1-12 | |
| A | WO 03/035904 A2 (CHRU TOURS [FR]; INNATE PHARMA [FR]; WATIER HERVE [FR]; CARTRON GUILLA) 1 May 2003 (2003-05-01) * the whole document * ----- | 1-12 | |
| A | AL-MASSARANI G ET AL: "Kidney transplantation decreases the level and procoagulant activity of circulating microparticles." AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS MAR 2009 LNKD- PUBMED:19260834, vol. 9, no. 3, March 2009 (2009-03), pages 550-557, XP002605895 ISSN: 1600-6143 * the whole document * ----- | 1-12 | TECHNICAL FIELDS SEARCHED (IPC) |

-/--

1

The present search report has been drawn up for all claims

| Place of search | Date of completion of the search | Examiner |
|---|---|---|
| Munich | 20 October 2010 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 35 8003

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEA RICHARD G ET AL: "Immunoendocrine aspects of endometrial function and implantation." REPRODUCTION (CAMBRIDGE, ENGLAND) SEP 2007 LNKD- PUBMED:17709558, vol. 134, no. 3, September 2007 (2007-09), pages 389-404, XP002605896 ISSN: 1470-1626 * the whole document * ----- | 1-12 | |

TECHNICAL FIELDS SEARCHED (IPC)

The present search report has been drawn up for all claims

1

| Place of search | Date of completion of the search | Examiner |
|---|---|---|
| Munich | 20 October 2010 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 35 8003

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2010

| Patent document cited in search report | Publication date | Patent family member(s) | Publication date |
|---|---|---|---|
| WO 03035904 A2 | 01-05-2003 | AT 403750 T | 15-08-2008 |
| | | CA 2463746 A1 | 01-05-2003 |
| | | CN 1571850 A | 26-01-2005 |
| | | DE 02782887 T1 | 13-01-2005 |
| | | DK 1436427 T3 | 08-12-2008 |
| | | EP 1436427 A2 | 14-07-2004 |
| | | ES 2311634 T3 | 16-02-2009 |
| | | JP 4216720 B2 | 28-01-2009 |
| | | JP 2005506093 T | 03-03-2005 |
| | | US 2005064417 A1 | 24-03-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683202 A
- US 5854033 A

### Non-patent literature cited in the description


- **D'HAESE et al.** *Expert opinion on therapeutic targets.,* 2010, vol. 14, 207-219
- **DAMAS et al.** *Arteriosclerosis, thrombosis, and vascular biology,* 2005, vol. 25, 2567-2572
- **UMEHARA et al.** *Arteriosclerosis, thrombosis, and vascular biology,* 2004, vol. 24, 34-40
- **BJERKELI et al.** *Rheumatology,* 2007, vol. 46, 1422-1427
- **STEPANIAN et al.** *PloS one,* 2009, vol. 4, e6192
- **BARANY.** *Proc. Natl. Acad sci USA,* 1991, vol. 88, 189-193
- **GUATELLI et al.** *Proc. Natl Acad Sci. USA,* 1990, vol. 87, 1874-1878
- **KWOH et al.** *Proc. Natl Acad Sci. USA,* 1989, vol. 86, 1173-1177
- **LIZARDI et al.** *Biol. Technology,* 1988, vol. 6, 1197